(19) Europäisches Patentamt / European Patent Office / Office européen des brevets

(11) **EP 1 894 930 A1**

(12) **EUROPEAN PATENT APPLICATION**
published in accordance with Art. 153(4) EPC

(43) Date of publication:
**05.03.2008 Bulletin 2008/10**

(21) Application number: **06780656.2**

(22) Date of filing: **23.06.2006**

(51) Int Cl.:
***C07D 417/14*** *(2006.01)* ***A61P 3/10*** *(2006.01)*
***A61P 9/08*** *(2006.01)* ***A61P 9/10*** *(2006.01)*
***A61P 21/00*** *(2006.01)* ***A61P 21/04*** *(2006.01)*
***A61P 25/00*** *(2006.01)* ***A61P 25/02*** *(2006.01)*
***A61P 25/06*** *(2006.01)* ***A61P 25/08*** *(2006.01)*
***A61P 25/14*** *(2006.01)* ***A61P 25/16*** *(2006.01)*
***A61P 25/18*** *(2006.01)* ***A61P 25/20*** *(2006.01)*
***A61P 25/22*** *(2006.01)* ***A61P 25/24*** *(2006.01)*
***A61P 25/28*** *(2006.01)* ***A61P 25/30*** *(2006.01)*

(86) International application number:
**PCT/JP2006/312619**

(87) International publication number:
**WO 2006/137527 (28.12.2006 Gazette 2006/52)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **23.06.2005 JP 2005183997**

(71) Applicant: **KYOWA HAKKO KOGYO CO., LTD.**
**Chiyoda-ku,**
**Tokyo 100-8185 (JP)**

(72) Inventors:
- **SUGAWARA, Masamori**
**c/o Pharmaceutical Research Center,**
**Nagaizumi-cho Sunto-gun Shizuoka 411 8731 (JP)**
- **NAKAJIMA, Takao**
**c/o Pharmaceutical Research Center,**
**Nagaizumi-cho Sunto-gun Shizuoka 411 8731 (JP)**
- **YAMADA, Koji**
**c/o Pharmaceutical Research Center,**
**Nagaizumi-cho Sunto-gun Shizuoka 411 8731 (JP)**
- **NAKASATO, Yoshisuke**
**c/o Pharmaceutical Research Center,**
**Nagaizumi-cho Sunto-gun Shizuoka 411 8731 (JP)**
- **UESAKA, Noriaki**
**c/o Pharmaceutical Research Center,**
**Nagaizumi-cho Sunto-gun Shizuoka 411 8731 (JP)**

(74) Representative: **Vossius & Partner**
**Siebertstrasse 4**
**81675 München (DE)**

# (54) THIAZOLE DERIVATIVE

(57) An adenosine $A_{2A}$ receptor antagonist comprising, as an active ingredient, a thiazole derivative represented by the general formula (I) [wherein $R^1$ represents a substituted or unsubstituted 5-membered aromatic heterocyclic group containing at least one oxygen atom; $R^2$ represents a substituted or unsubstituted heterocyclic group, $-COR^6$ (Wherein $R^6$ represents a substituted or unsubstituted heterocyclic group, a substituted or unsubstituted aromatic heterocyclic group, or the like), or the like; and $R^3$ represents the general formula (II), $-NHCOR^7$ (wherein $R^7$ represents $-NR^8R^9$, or the like) or the like] or a pharmaceutically acceptable salt thereof, and the like are provided,

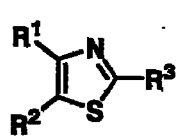
(I)

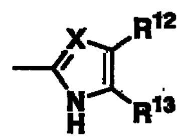
(II)

EP 1 894 930 A1

## Description

Technical Field

The present invention relates to, for example, thiazole derivatives or pharmaceutically acceptable salts thereof which have an adenosine $A_{2A}$ receptor antagonism, etc.

Background Art

It is known that adenosine ranges broadly in a living body and exhibits various physiological actions on the central nervous system, the cardiac muscle, the kidney, the lung, the smooth muscle or the like via its receptor. Four subtypes of adenosine receptors, $A_1$, $A_{2A}$, $A_{2B}$ and $A_3$ have heretofore been known. The respective subtypeselective receptor antagonists and agonists are expected to exhibit their pharmacological effects based on the physiological meanings of the subtype and on the biological distribution thereof. Among them, the $A_{2A}$ receptors are localized in the brain, especially in the corpus striatum thereof, and as one of its functions, the inhibition of neurotransmitter release is reported (European Journal of Pharmacology, Vol. 168, p. 285, 1989). Accordingly, antagonists to the adenosine $A_{2A}$ receptor may be expected as therapeutic and/or preventive agents for diseases associated with adenosine $A_{2A}$ receptor, such as central nervous system diseases including Parkinson's disease, Alzheimer's disease, progressive supranuclear palsy, AIDS encephalopathy, transmissible spongiform encephalopathy, multiple sclerosis, amyotrophic lateral sclerosis, Huntington's disease, multiple system atrophy, cerebral ischemia, attention deficit hyperactivity disorder, sleep disorder, intermittent claudication, diabetes, anxiety disorders (such as panic attack and panic disorder, phobia, obsessive-compulsive disorder, posttraumatic stress disorder, acute stress disorder, generalized anxiety disorder, and anxiety caused by physical disorder or a substance), mood disorders (such as depression, dysthymic disorder, and mood-circulatory disorder), restless legs syndrome (RLS), drug dependence (such as alcohol dependence), eating disorder, epilepsy, migraine and chronic musculoskeletal pain or the like, and ischemic heart diseases including myocardial infarction, cerebral infarction or the like.

On the other hand, a large number of compounds having a thiazole skeleton are known (for example, see Patent documents 1 to 23 and 35 to 53, etc.). For example, thiazole derivatives having an adenosine $A_3$ receptor antagonism (see Patent documents 24 and 25), thiazole derivatives having an adenosine $A_{2B}$ receptor antagonism and an adenosine $A_3$ receptor antagonism (see Patent documents 26 and 27), and thiazole derivatives having an adenosine $A_1$ receptor antagonism and an adenosine $A_{2A}$ receptor antagonism (see Patent documents 28 and 29) and the like are known.

Further, thiazole derivatives having a furyl group at the 4 position thereof are known (see Patent documents 30 to 34 and Non-patent documents 1 to 5).

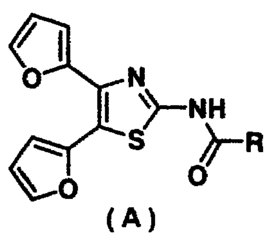

(A)

(wherein R represents phenylmethyl, 2-furyl, 4-fluorophenyl, 2-fluorophenyl, 2,4-dichlorophenyl, 4-nitrophenyl, 2-nitrophenyl, 4-bromophenyl, 3-bromophenyl, 2-bromophenyl, 2-chlorophenyl, 3-bromo-2-methoxyphenyl, 4-tert-butylphenyl, 3-methylphenyl, 4-methylphenyl, 4-methoxyphenyl, 2-methoxyphenyl or phenyl.)

Also, thiazole derivatives represented by the above-mentioned general formula (A) are known (see Non-patent document 6).

Further, thiazole derivatives having a carbonyl group at the 5 position thereof are known (see Patent document 29 and Patent documents 35 to 42).

Patent document 1: US Patent No. 5,314,889

Patent document 2: US Patent No. 5,189,049

Patent document 3: Japanese Published Unexamined Patent Application No. 335680/2003

Patent document 4: Japanese Published Unexamined Patent Application No. 53566/2002

Patent document 5: Japanese Published Unexamined Patent Application No. 209284/1999

Patent document 6: Japanese Published Unexamined Patent Application No. 087490/1998
Patent document 7: WO93/21168
Patent document 8: WO96/16650
Patent document 9: WO97/03058
Patent document 10: WO01/52847
Patent document 11: WO01/53267
Patent document 12: WO01/74811
Patent document 13: WO02/053156
Patent document 14: WO02/053161
Patent document 15: WO02/094798
Patent document 16: WO03/000257
Patent document 17: WO03/062215
Patent document 18: WO03/062233
Patent document 19: WO03/072554
Patent document 20: WO03/075923

Patent document 21: W02004/002481
Patent document 22: W02004/014884
Patent document 23: W02004/041813
Patent document 24: WO99/21555
Patent document 25: Japanese Published Unexamined Patent Application No. 114779/2001
Patent document 26: WO99/64418
Patent document 27: US Patent Application Publication No. 2004-0053982
Patent document 28: WO03/039451
Patent document 29: W02005/039572
Patent document 30: US Patent No. 6,489,476
Patent document 31: WO02/03978
Patent document 32: WO01/47935
Patent document 33: WO00/38666
Patent document 34: WO00/14095
Patent document 35: Japanese Published Unexamined Patent Application No. 079993/1999
Patent document 36: WO95/29904
Patent document 37: WO98/57937
Patent document 38: WO00/57877
Patent document 39: WO02/083111
Patent document 40: WO03/040147

Patent document 41: WO2004/016622
Patent document 42: W02004/094395
Patent document 43: Japanese Published Unexamined Patent Application No. 280255/1988
Patent document 44: WO97/12615
Patent document 45: Japanese Published Unexamined Patent Application No. 302680/1993
Patent document 46: WO01/10865
Patent document 47: WO01/14372
Patent document 48: WO02/051442
Patent document 49: WO02/100433
Patent document 50: Japanese Published Unexamined Patent Application No. 345772/1993
Patent document 51: WO02/059099
Patent document 52: WO01/74811
Patent document 53: WO99/21555

Non-patent document 1: Chemistry of Heterocyclic Compounds, Vol. 38, p.873, 2002
Non-patent document 2: Khimiko-Farmatsevticheskii Zhurnal, Vol. 8, p.25, 1974
Non-patent document 3: Journal of Medicinal Chemistry, Vol. 13, p.638, 1970
Non-patent document 4: Khimiya Geterotsiklicheskikh Soedinenii, Vol. 3, p.498, 1969
Non-patent document 5: Journal of Organic Chemistry, Vol. 27, p.1351, 1962

Non-patent document 6: CAS REGISTRY Database, Registry Nos.: 341929-13-3, 341929-11-1, 341929-09-7, 341929-07-5, 341929-05-3, 341929-04-2, 341929-02-0, 341929-00-8, 341928-98-1, 341928-96-9, 341928-94-7, 341928-92-5, 341928-90-3, 341928-88-9, 341928-86-7, 341928-84-5, 341928-82-3, 341928-80-1, 757215-26-2, 756865-67-5, 756843-07-9, 756840-57-0, 753474-31-6, 752227-21-7, 750629-53-9, 750629-34-6, 750622-46-9, 750610-49-2, 749913-85-7, 749905-39-3, 749888-72-0, 749210-08-0, 747409-81-0, 736952-04-8, 732271-25-9,

731014-24-7, 730996-54-0, 730950-78-4, 730942-51-5, 729580-28-3, 727716-11-2, 727697-43-0, 722472-19-7, 721913-95-7, 721899-36-1, 702636-14-4, 568543-98-6, 554425-13-7, 681237-56-9, 477554-77-1, 477554-76-0, 477554-75-9, 477554-74-8, 476642-94-1, 476641-16-4, 476355-73-4, 476317-07-4, 476281-51-3, 476209-54-8, 475043-45-9, 475043-44-8, 475043-43-7, 393837-57-5, 392325-20-1, 392251-67-1, 392246-46-7, 392238-65-2, 392237-01-3, 391225-59-5, 391223-92-0, 391222-36-9, 391220-46-5, 361480-79-7, 361166-65-6, 361159-23-1, 330201-67-7, 330201-44-0, 329905-74-0, 329905-51-3, 329903-27-7, 329903-22-2, 329903-20-0, 328038-44-4, 326609-62-5, 326025-09-6, 325831-63-8, 324541-23-3, 324541-22-2, 324541-21-1, 324541-20-0, 324541-19-7, 313274-43-0, 313274-42-9, 313274-41-8, 312614-29-2, 312614-28-1, 312614-27-0, 306289-61-2, 782452-94-2, 769928-58-7, 752986-14-4, 679802-30-3, 677780-72-2, 477536-94-0, 477536-67-7, 477536-42-8, 477534-53-5, 477508-22-8, 477508-21-7, 477508-20-6, 477508-19-3, 477507-05-4, 477507-04-3, 477507-03-2, 477507-02-1, 477507-01-0, 477496-55-2, 477496-21-2, 477495-79-7, 477495-77-5, 477495-75-3, 477495-73-1, 477495-21-9, 477494-88-5, 477491-93-3, 477491-55-7, 477324-67-7, 477323-97-0, 477323-76-5, 477323-19-6, 477323-05-0, 477322-55-7, 477322-40-0, 477311-94-7, 477311-93-6, 477311-91-4, 477311-89-0, 476356-23-7, 476356-10-2, 476307-77-4, 476279-40-0, 476274-91-6, 398998-39-5, 396724-86-0, 391896-51-8, 790274-61-2, 786716-88-9, 765283-91-8, 743464-46-2, 743464-39-3 and 722467-73-4

Disclosure of the Invention

Problem to be Solved by the Invention

An object of the present invention is to provide, for example, thiazole derivatives or pharmaceutically acceptable salts thereof which have an adenosine $A_{2A}$ receptor antagonism and are useful for treating and/or preventing various diseases associated with adenosine $A_{2A}$ receptor (e.g., Parkinson's disease, Alzheimer's disease, depression, an ischemic disease such as cerebral infarction or myocardial infarction, etc.) and the like.

Means for Solving the Problem

The present invention relates to the following (1) to (23).

(1) An adenosine $A_{2A}$ receptor antagonist comprising, as an active ingredient, a thiazole derivative represented by the general formula (I):

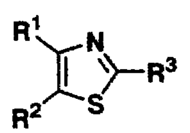 (I)

{wherein ;
$R^1$ represents a substituted or unsubstituted 5-membered aromatic heterocyclic group containing at least one oxygen atom;
$R^2$ represents halogen,
substituted or unsubstituted lower alkyl,
substituted or unsubstituted lower alkenyl,
substituted or unsubstituted lower alkynyl,
substituted or unsubstituted cycloalkyl,
substituted or unsubstituted aryl,
substituted or unsubstituted aralkyl,
a substituted or unsubstituted alicyclic heterocyclic group,
a substituted or unsubstituted aromatic heterocyclic group,
substituted or unsubstituted alicyclic heterocyclic-alkyl,
substituted or unsubstituted aromatic heterocyclic-alkyl,
$-NR^4R^5$ (wherein
$R^4$ and $R^5$ may be the same or different, and each represents
a hydrogen atom,
substituted or unsubstituted lower alkyl,

substituted or unsubstituted lower alkenyl,
substituted or unsubstituted lower alkynyl,
substituted or unsubstituted lower alkanoyl,
substituted or unsubstituted cycloalkyl,
substituted or unsubstituted aryl,
substituted or unsubstituted aralkyl,
a substituted or unsubstituted alicyclic heterocyclic group,
a substituted or unsubstituted aromatic heterocyclic group,
substituted or unsubstituted alicyclic heterocyclic-alkyl, or
substituted or unsubstituted aromatic heterocyclic-alkyl), or
-COR$^6$ (wherein
R$^6$ represents substituted or unsubstituted lower alkyl,
substituted or unsubstituted lower alkenyl,
substituted or unsubstituted lower alkynyl,
substituted or unsubstituted cycloalkyl,
substituted or unsubstituted aryl,
substituted or unsubstituted aralkyl,
a substituted or unsubstituted alicyclic heterocyclic group,
a substituted or unsubstituted aromatic heterocyclic group,
substituted or unsubstituted alicyclic heterocyclic-alkyl, or
substituted or unsubstituted aromatic heterocyclic-alkyl): and

R$^3$ represents -NHCOR$^7$ [wherein
R$^7$ represents -NR$^8$R$^9$ (wherein
R$^8$ and R$^9$ may be the same or different, and each represents
a hydrogen atom,
substituted or unsubstituted lower alkyl,
substituted or unsubstituted lower alkenyl,
substituted or unsubstituted lower alkynyl,
substituted or unsubstituted lower alkanoyl,
substituted or unsubstituted lower alkoxy,
substituted or unsubstituted cycloalkyl,
substituted or unsubstituted aryl,
substituted or unsubstituted aralkyl,
substituted or unsubstituted aroyl,
a substituted or unsubstituted alicyclic heterocyclic group,
a substituted or unsubstituted aromatic heterocyclic group,
substituted or unsubstituted alicyclic heterocyclic-alkyl, or
substituted or unsubstituted aromatic heterocyclic-alkyl, or
R$^8$ and R$^9$ are combined together with the adjacent nitrogen atom thereto to form a substituted or unsubstituted alicyclic heterocyclic group) or
-OR$^{10}$ (wherein
R$^{10}$ represents substituted or unsubstituted lower alkyl,
substituted or unsubstituted lower alkenyl,
substituted or unsubstituted lower alkynyl,
substituted or unsubstituted cycloalkyl,
substituted or unsubstituted aryl,
substituted or unsubstituted aralkyl,
a substituted or unsubstituted alicyclic heterocyclic group, or
a substituted or unsubstituted aromatic heterocyclic group,
substituted or unsubstituted alicyclic heterocyclic-alkyl,
substituted or unsubstituted aromatic heterocyclic-alkyl)],
-CONHR$^{11}$ (wherein
R$^{11}$ represents a hydrogen atom,
substituted or unsubstituted lower alkyl,
substituted or unsubstituted lower alkenyl,
substituted or unsubstituted lower alkynyl,
substituted or unsubstituted lower alkanoyl,
substituted or unsubstituted cycloalkyl,

substituted or unsubstituted aryl,
substituted or unsubstituted aralkyl,
substituted or unsubstituted aroyl,
a substituted or unsubstituted alicyclic heterocyclic group,
a substituted or unsubstituted aromatic heterocyclic group,
substituted or unsubstituted alicyclic heterocyclic-alkyl, or
substituted or unsubstituted aromatic heterocyclic-alkyl), or
the general formula (II):

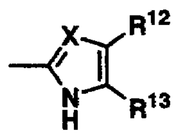

(II)

(wherein
=X- represents =CH- or =N-,
$R^{12}$ and $R^{13}$ may be the same or different, and each represents
a hydrogen atom,
halogen,
hydroxy,
nitro,
azido,
amino,
cyano,
carboxy,
formyl,
substituted or unsubstituted lower alkyl,
substituted or unsubstituted lower alkenyl,
substituted or unsubstituted lower alkynyl,
substituted or unsubstituted lower alkanoyl,
substituted or unsubstituted lower alkoxy,
substituted or unsubstituted cycloalkyl,
substituted or unsubstituted lower alkylamino,
substituted or unsubstituted di-lower alkylamino,
substituted or unsubstituted aryl,
substituted or unsubstituted aralkyl,
substituted or unsubstituted aroyl,
a substituted or unsubstituted alicyclic heterocyclic group,
a substituted or unsubstituted aromatic heterocyclic group,
substituted or unsubstituted alicyclic heterocyclic-alkyl, or
substituted or unsubstituted aromatic heterocyclic-alkyl, or
$R^{12}$ and $R^{13}$ are combined with the two carbon atoms adjacent thereto, respectively, to form
a substituted or unsubstituted carbon ring, or
a substituted or unsubstituted heterocyclic ring)} or a pharmaceutically acceptable salt thereof.

(2) The adenosine $A_{2A}$ receptor antagonist according to the above (1), wherein $R^1$ is substituted or unsubstituted furyl.

(3) An agent for preventing and/or treating a disease associated with adenosine $A_{2A}$ receptor comprising, as an active ingredient, the thiazole derivative or the pharmaceutically acceptable salt thereof described in the above (1) or (2).

(4) A thiazole derivative represented by the general formula (IA):

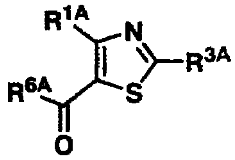

(I A)

(wherein $R^{1A}$, $R^{3A}$ and $R^{6A}$ have the same meanings as $R^1$, $R^3$ and $R^6$ defined above, respectively) or a pharmaceutically acceptable salt thereof.

(5) The thiazole derivative or the pharmaceutically acceptable salt thereof according to the above (4), wherein $R^{1A}$ is substituted or unsubstituted furyl.

(6) The thiazole derivative or the pharmaceutically acceptable salt thereof according to the above (4) or (5), wherein $R^{6A}$ is a substituted or unsubstituted alicyclic heterocyclic group, or a substituted or unsubstituted aromatic heterocyclic group, and -CO- in -$COR^{6A}$ bonds to a carbon atom of $R^{6A}$.

(7) The thiazole derivative or the pharmaceutically acceptable salt thereof according to any one of the above (4) to (6), wherein $R^{6A}$ is a substituted or unsubstituted alicyclic heterocyclic group.

(8) The thiazole derivative or the pharmaceutically acceptable salt thereof according to any one of the above (4) to (6), wherein $R^{6A}$ is substituted or unsubstituted tetrahydropyranyl, substituted or unsubstituted pyridyl or substituted or unsubstituted dioxepanyl.

(9) The thiazole derivative or the pharmaceutically acceptable salt thereof according to any one of the above (4) to (8), wherein $R^{3A}$ is -$CONHR^{11}$ (wherein $R^{11}$ has the same meaning as defined above).

(10) The thiazole derivative or the pharmaceutically acceptable salt thereof according to any one of the above (4) to (8), wherein $R^{3A}$ is the formula (II):

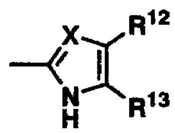

(II)

(wherein X, $R^{12}$ and $R^{13}$ have the same meanings as defined above, respectively).

(11) The thiazole derivative or the pharmaceutically acceptable salt thereof according to the above (10), wherein $R^{12}$ and $R^{13}$ are combined with the two carbon atoms adjacent thereto, respectively, to form a substituted or unsubstituted carbon ring, or a substituted or unsubstituted heterocyclic ring.

(12) The thiazole derivative or the pharmaceutically acceptable salt thereof according to any one of the above (4) to (8), wherein $R^{3A}$ is -$NHCOR^7$ (wherein $R^7$ has the same meaning as defined above).

(13) A pharmaceutical composition comprising, as an active ingredient, the thiazole derivative or the pharmaceutically acceptable salt thereof described in any one of the above (4) to (12).

(14) An adenosine $A_{2A}$ receptor antagonist comprising, as an active ingredient, the thiazole derivative or the pharmaceutically acceptable salt thereof described in any one of the above (4) to (12).

(15) An agent for preventing and/or treating a disease associated with adenosine $A_{2A}$ receptor comprising, as an active ingredient, the thiazole derivative or the pharmaceutically acceptable salt thereof described in any one of the above (4) to (12).

(16) A method for antagonizing adenosine $A_{2A}$ receptor comprising the step of administering an effective amount of the thiazole derivative or the pharmaceutically acceptable salt thereof described in the above (1) or (2).
(17) A method for preventing and/or treating a disease associated with adenosine $A_{2A}$ receptor comprising the step of administering an effective amount of the thiazole derivative or the pharmaceutically acceptable salt thereof described in the above (1) or (2).
(18) A method for antagonizing adenosine $A_{2A}$ receptor comprising the step of administering an effective amount of the thiazole derivative or the pharmaceutically acceptable salt thereof described in any one of the above (4) to (12).
(19) A method for preventing and/or treating a disease associated with adenosine $A_{2A}$ receptor comprising the step of administering an effective amount of the thiazole derivative or the pharmaceutically acceptable salt thereof described in any one of the above (4) to (12).

(20) Use of the thiazole derivative or the pharmaceutically acceptable salt thereof described in the above (1) or (2) for the manufacture of an adenosine $A_{2A}$ receptor antagonist.
(21) Use of the thiazole derivative or the pharmaceutically acceptable salt thereof described in the above (1) or (2) for the manufacture of an agent for preventing and/or treating a disease associated with adenosine $A_{2A}$ receptor.
(22) Use of the thiazole derivative or the pharmaceutically acceptable salt thereof described in any one of the above (4) to (12) for the manufacture of an adenosine $A_{2A}$ receptor antagonist.
(23) Use of the thiazole derivative or the pharmaceutically acceptable salt thereof described in any one of the above (4) to (12) for the manufacture of an agent for preventing and/or treating a disease associated with adenosine $A_{2A}$ receptor.

Effect of the Invention

According to the present invention, for example, thiazole derivatives or pharmaceutically acceptable salts thereof which have an adenosine $A_{2A}$ receptor antagonism and are useful for treating and/or preventing various diseases associated with adenosine $A_{2A}$ receptor (e.g., Parkinson's disease, Alzheimer's disease, depression, an ischemic disease such as cerebral infarction or myocardial infarction, etc.) and the like are provided.

Best Mode for Carrying Out the Invention

Hereinafter, the compounds represented by the general formulae (I) and (IA) are referred to as Compounds (I) and (IA), and the same applies to compounds of other formula numbers. In the definition of each group in the general formulae (I) and (IA):

(i) Examples of the lower alkyl and the lower alkyl moiety of the lower alkanoyl, the lower alkoxy, the lower alkylamino and the di-lower alkylamino include linear or branched alkyl having 1 to 10 carbon atoms, such as methyl, ethyl, propyl, isopropyl, butyl, isobutyl, sec-butyl, tert-butyl, pentyl, isopentyl, neopentyl, hexyl, heptyl, octyl, nonyl and decyl.

The two lower alkyl moieties of the di-lower alkylamino may be the same or different.

(ii) Examples of the lower alkenyl include linear or branched alkenyl having 2 to 10 carbon atoms, such as vinyl, allyl, 1-propenyl, butenyl, pentenyl, hexenyl, heptenyl, octenyl, nonenyl and decenyl.
(iii) Examples of the lower alkynyl include linear or branched alkynyl having 2 to 10 carbon atoms, such as ethynyl, propynyl, butynyl, pentynyl, hexynyl, heptynyl, octynyl, nonynyl and decynyl.
(iv) Examples of the cycloalkyl include cycloalkyl having 3 to 8 carbon atoms, bridged cyclic hydrocarbon groups having 4 to 8 carbon atoms, bicyclic or tricyclic spiro hydrocarbon groups in which cycloalkyl having 3 to 8 carbon atoms are spiro-linked and the like, such as cyclopropyl, cyclobutyl, cyclopentyl, cyclohexyl, cycloheptyl, cyclooctyl, adamantyl, noradamantyl, bicyclo[2.2.1]heptyl, and spiro[4.5]decanyl.

(v) Examples of the aryl and the aryl moiety of the aralkyl and the aroyl include aryl having 6 to 10 carbon atoms, such as phenyl and naphthyl.
(vi) Examples of the aromatic heterocyclic group and the aromatic heterocyclic moiety of the aromatic heterocyclic-alkyl include 5- or 6-membered monocyclic aromatic heterocyclic groups containing at least one atom selected from a nitrogen atom, an oxygen atom and a sulfur atom, bicyclic or tricyclic fused-ring aromatic heterocyclic groups containing at least one atom selected from a nitrogen atom, an oxygen atom and a sulfur atom in which 4- to 8-membered rings are fused and the like, such as furyl, thienyl, pyrrolyl, pyridyl, N-oxopyridyl, pyrazinyl, imidazolyl, pyrazolyl, triazolyl, thiazolyl, isothiazolyl, thiadiazolyl, oxazolyl, isoxazolyl, oxadiazolyl, pyrimidinyl, pyridazinyl, indolyl, isoindolyl, benzothienyl, benzofuranyl, benzothiazolyl, benzimidazolyl, benzothiadiazolyl, benzotriazolyl, quinolyl, isoquinolyl, quinazolinyl and furo[2,3-b]pyridyl.

(vii) Examples of the 5-membered aromatic heterocyclic group containing at least one oxygen atom include the 5-membered monocyclic aromatic heterocyclic groups containing at least one oxygen atom of the 5-membered monocyclic aromatic heterocyclic groups described in the above examples of the aromatic heterocyclic group (vi), such as furyl, oxazolyl, isoxazolyl and oxadiazolyl.

(viii) Examples of the alicyclic heterocyclic group and the alicyclic heterocyclic moiety of the alicyclic heterocyclic-alkyl include 3- to 8-membered monocyclic alicyclic heterocyclic groups containing at least one atom selected from a nitrogen atom, an oxygen atom and a sulfur atom, bicyclic or tricyclic fused-ring alicyclic heterocyclic groups containing at least one atom selected from a nitrogen atom, an oxygen atom and a sulfur atom in which 4- to 8-membered rings are fused, 5- to 10-membered bridged cyclic heterocyclic groups, containing two bridgehead carbon atoms and at least one atom selected from a nitrogen atom, an oxygen atom and a sulfur atom, being bridged through said two bridgehead carbon atoms, bicyclic or tricyclic spiro alicyclic heterocyclic groups containing at least one atom selected from a nitrogen atom, an oxygen atom and a sulfur atom in which 4- to 8-membered rings are spiro-linked, and the like, such as pyrrolidinyl, imidazolidinyl, thiazolidinyl, piperidyl, piperazinyl, morpholinyl, thiomorpholinyl, piperidino, morpholino, thiomorpholino, oxazolinyl, dioxolanyl, dioxanyl, dioxepanyl, dihydropyridyl, tetrahydropyridyl, pyranyl, dihydropyranyl, tetrahydropyranyl, tetrahydroisoquinolyl, tetrahydroquinolyl, indolinyl, isoindolinyl, octahydropyrazino[2,1-c] [1,4]oxazinyl, dihydropyridazinyl, oxiranyl, oxetanyl, oxolanyl, thiolanyl, thianyl, aziridinyl, azetidinyl, azolidinyl, perhydrooxazepinyl, perhydrothiazepinyl, perhydroazepinyl, perhydroazocinyl, perhydrodiazepinyl, succinimido, phthalimido, glutarimido, 1,3-benzodioxolyl, 1,4-benzodioxanyl, 3,4-dihydro-2H-1,5-benzodioxepinyl, 1,4-dioxaspiro[4.5]decanyl, 1,4-dioxa-8-azaspiro[4.5]decanyl, octahydropyrrolo[1,2-a]pyrazinyl, octahydropyrazino[2,1-c][1,4]oxazinyl, octahydropyrazino[2,1-c][1,4]thiazinyl, 1-oxaspiro[4.5]decanyl, 1-oxa-8-azaspiro[4.5]decanyl, 8-oxa-3-azabicyclo[3.2.1]octanyl, 7-oxa-bicyclo[2.2.1]heptyl, and 2-oxa-5-azabicyclo[2.2.1]heptyl.

(ix) Examples of the alkylene moiety of the aralkyl, the aromatic heterocyclic-alkyl and the alicyclic heterocyclic-alkyl include linear or branched alkylene having 1 to 10 carbon atoms, such as one produced by removing one hydrogen atom from each of the groups described in the above examples of the lower alkyl (i).

(x) The halogen means each atom of fluorine, chlorine, bromine and iodine.

(xi) Examples of the alicyclic heterocyclic group formed together with the adjacent nitrogen atom include 3-to 8-membered monocyclic alicyclic heterocyclic groups containing at least one nitrogen atom, bicyclic or tricyclic fused-ring alicyclic heterocyclic groups containing at least one nitrogen atom, in which 4- to 8-membered rings are fused, 5- to 10-membered bridged cyclic heterocyclic groups, containing at least one nitrogen atom and two bridgehead carbon atoms, being bridged through said two bridgehead carbon atoms bicyclic or tricyclic spiro alicyclic heterocyclic groups containing at least one nitrogen atom, in which 4- to 8-membered rings are spiro-linked, and the like, which may further containing atoms selected from a nitrogen atom, an oxygen atom and a sulfur atom, such as pyrrolidinyl, oxazolinyl, piperidino, perhydroazepinyl, piperazinyl, morpholino, thiomorpholino, homopiperazinyl, dihydropyridyl, tetrahydropyridyl, tetrahydropyrazinyl, tetrahydroquinolyl, tetrahydroisoquinolyl, indolinyl, isoindolinyl, octahydropyrazino[2,1-c][1,4]oxazinyl, dihydropyridazinyl, aziridinyl, azetidinyl, azolidinyl, perhydrooxazepinyl, perhydrothiazepinyl, perhydroazepinyl, perhydroazocinyl, perhydrodiazepinyl, 1,4-dioxa-8-azaspiro[4.5]decanyl, octahydropyrrolo[1,2-a]pyrazinyl, octahydropyrazino[2,1-c][1,4]oxazinyl octahydropyrazino[2,1-c][1,4]thiazinyl, 1-oxaspiro[4.5]decanyl, 1-oxa-8-azaspiro[4.5]decanyl, 8-oxa-3-azabicyclo[3.2.1]octanyl, 2-oxa-5-azabicyclo[2.2.1]heptyl, and 8-oxa-3-azabicyclo[3.2.1]octanyl.

(xii) Examples of the carbon ring formedtogether with the two adjacent carbon atoms include 3- to 8-membered alicyclic or aromatic carbon rings, such as cyclopropene, cyclobutene, cyclopentene, cyclohexene, cycloheptene,

cyclooctene and benzene. A double bond is formed between a pair of adjacent two carbon atoms of the alicyclic carbon ringwherein hydrogen atoms on the carbon atoms are eliminated.

(xiii) Examples of the heterocyclic ringformed together with the two adjacent carbon atoms include 3- to 8-membered alicyclic or aromatic heterocyclic rings, specific examples thereof include 3- to 8-membered monocyclic alicyclic or aromatic heterocyclic rings containing at least one atom selected from a nitrogen atom, an oxygen atom and a sulfur atom, bicyclic or tricyclic fused-ring alicyclic or aromatic heterocyclic rings containing at least one atom selected from a nitrogen atom, an oxygen atom and a sulfur atom, in which 4- to 8-membered rings are fused, such as pyrrolidine, imidazolidine, thiazolidine, piperidine, piperazine, morpholine, thiomorpholine, oxazoline, dioxolane, dioxane, dioxepane, dihydropyridine, tetrahydropyridine, pyran, dihydropyran, tetrahydropyran, tetrahydroisoquinoline, tetrahydroquinoline, thiopyran, dihydrothiopyran, tetrahydrothiopyran, indoline, isoindoline, octahydropyrazino [2,1-c][1,4]oxazine, dihydropyridazine, oxirane, oxetane, oxorane, thiorane, thiane, aziridine, azetidine, azolidine, perhydrooxazepine, perhydrothiazepine, perhydroazepine, perhydroazocine, perhydrodiazepine, succinimide, phthalimide, glutarimide, 1,3-benzodioxone, 1,4-benzodioxane, and 3,4-dihydro-2H-1,5-benzodioxepine, which are obtained by eliminating hydrogen atoms on the respective two adjacent carbon atoms of alicyclic heterocyclic rings from the alicyclic heterocyclic rings, and aromatic heterocyclic rings , such as furan, thiophene, pyrrole, pyridine, N-oxopyridine, pyrazine, imidazole, pyrazole, triazole, thiazole, isothiazole, thiadiazole, oxazole, isoxazole, oxadiazole, pyrimidine, pyridazine, indoline, isoindoline, benzothiophene, benzofuran, benzothiazole, benzoimidazole, benzothiadiazole, benzotriazole, quinoline, isoquinoline, and quinazoline.

(xiv) Examples of the substituents (A) in the substituted lower alkyl include 1 to 3 substituents which may be the same or different, such as halogen, hydroxy, nitro, azido, amino, cyano, carboxy, formyl, substituted or unsubstituted lower alkoxy, substituted or unsubstituted lower alkanoyloxy, substituted or unsubstituted lower alkylsulfanyl, substituted or unsubstituted lower alkylamino, substituted or unsubstituted di-lower alkylamino, substituted or unsubstituted adamantylamino, substituted or unsubstituted cycloalkyl, substituted or unsubstituted aryloxy and substituted or unsubstituted heterocyclic-oxy.

In the examples of the substituents (A), examples of the substituents (a) in the substituted lower alkoxy, the substituted lower alkanoyloxy, the substituted lower alkylsulfanyl, the substituted lower alkylamino and the substituted di-lower alkylamino include 1 to 3 substituents which may be the same or different, such as halogen, hydroxy, hydroxyimino, methoxyimino, nitro, azido, amino, cyano, carboxy, cycloalkyl, substituted or unsubstituted lower alkoxy, substituted or unsubstituted substituted lower alkanoyloxy, substituted or unsubstituted lower alkylamino, substituted or unsubstituted di-lower alkylamino, substituted or unsubstituted aryl, a substituted or unsubstituted alicyclic heterocyclic group, a substituted or unsubstituted aromatic heterocyclic group, aryloxy and heterocyclic-oxy.

In the examples of the substituents (a), examples of the substituents (b) in the substituted lower alkoxy, the substituted lower alkanoyloxy, the substituted lower alkylamino and the substituted di-lower alkylamino include 1 to 3 substituents which may be the same or different, such as halogen, hydroxy, amino, lower alkoxy, lower alkylamino, di-lower alkylamino, aryl, an alicyclic heterocyclic group and an aromatic heterocyclic group.

In the examples of the substituents (a), examples of the substituents (c) in the substituted aryl and the substituted aromatic heterocyclic group include 1 to 3 substituents which may be the same or different, such as the groups described in the examples of the substituents (b), and lower alkyl.

In the examples of the substituents (a), examples of the substituents (d) in the substituted alicyclic heterocyclic group include 1 to 3 substituents which may be the same or different, such as the groups described in the examples of the substituents (b), lower alkyl and oxo.

In the examples of the substituents (A), examples of the substituents (e) in the substituted adamantylamino include 1 to 3 substituents which may be the same or different, such as lower alkyl, lower alkoxy, hydroxy, oxo and formyl.

In the examples of the substituents (A), examples of the substituents (f) in the substituted cycloalkyl include 1 to 3 substituents which may be the same or different, such as the groups described in the examples of the substituents (a), lower alkyl, oxo and formyl.

In the examples of the substituents (A), examples of the substituents (g) in the substituted aryloxy and the substituted heterocyclic-oxy include 1 to 3 substituents which may be the same or different, such as halogen, hydroxy, nitro, azido, amino, cyano, carboxy, formyl, substituted or unsubstituted lower alkyl, substituted or unsubstituted lower alkanoyl, cycloalkyl, lower alkoxy, lower alkylsulfanyl, lower alkylsulfonyl, lower alkoxycarbonyl, lower alkylaminocarbonyl, di-lower alkylaminocarbonyl, lower alkylamino, di-lower alkylamino, aryl, an alicyclic heterocyclic group, an aromatic heterocyclic group, aryloxy and heterocyclic-oxy.

In the examples of the substituents (g), examples of the substituents in the substituted lower alkyl and the substituted lower alkanoyl include 1 to 3 substituents which may be the same or different, such as the groups described

in the examples of the substituents (b).
In the examples of the substituents (A), the substituents (a), the substituents (b), the substituents (c), the substituents (d), the substituents (e), the substituents (f) and the substituents (g), examples of the lower alkyl moiety of the lower alkyl, the lower alkoxy, the lower alkanoyl, the lower alkanoyloxy, the lower alkylsulfanyl, the lower alkylsulfonyl, the lower alkoxycarbonyl, the lower alkylaminocarbonyl, the di-lower alkylaminocarbonyl, the lower alkylamino and the di-lower alkylamino; the cycloalkyl; the aryl moiety of the aryl and the aryloxy; the alicyclic heterocyclic group; the aromatic heterocyclic group; and the halogen have the same meanings as the lower alkyl (i), the cycloalkyl (iv), the aryl (v), the alicyclic heterocyclic group (viii), the aromatic heterocyclic group (vi) and the halogen (x) defined above, respectively; examples of the heterocyclic moiety of the heterocyclic-oxy include the groups described in the above examples of the alicyclic heterocyclic group (viii), the aromatic heterocyclic group (vi) and the like; and the two lower alkyl moieties of the di-lower alkylaminocarbonyl and the di-lower alkylamino may be the same or different.

(xv) Examples of the substituents (B) in the substituted lower alkanoyl, the substituted lower alkenyl, the substituted lower alkynyl and the substituted lower alkoxy, the substituted lower alkylamino and the substituted di-lower alkylamino include 1 to 3 substituents which may be the same or different, such as the groups described in the examples of the substituents (A), substituted or unsubstituted aryl, a substituted or unsubstituted alicyclic heterocyclic group, and a substituted or unsubstituted aromatic heterocyclic group.

In the examples of the substituents (B), examples of the substituents (h) in the substituted aryl and the substituted aromatic heterocyclic group include 1 to 3 substituents which may be the same or different, such as the groups described in the examples of the substituents (a) and lower alkyl.
In the examples of the substituents (B), examples of the substituents (j) in the substituted alicyclic heterocyclic group include 1 to 3 substituents which may be the same or different, such as the groups described in the examples of the substituents (a), lower alkyl, oxo and formyl.
In the examples of the substituents (B), the substituents (h) and the substituents (j), the lower alkyl, the aryl, the alicyclic heterocyclic group and the aromatic heterocyclic group have the same meanings as the lower alkyl (i), the aryl (v), the alicyclic heterocyclic group (viii) and the aromatic heterocyclic group (vi) defined above, respectively.

(xvi) Examples of the substituents (C) in the substituted cycloalkyl and substituted carbon ring formed together with the two adjacent carbon atoms include 1 to 3 substituents which may be the same or different, such as the groups described in the examples of the substituents (A), lower alkyl and oxo.

In the examples of the substituents (C), the lower alkyl has the same meaning as the lower alkyl (i) defined above.

(xvii) Examples of the substituents (D) in the substituted aryl, the substituted aralkyl, the substituted aroyl, the substituted aromatic heterocyclic group, the substituted pyridyl, the substituted 5-membered aromatic heterocyclic group containing at least one oxygen atom, the substituted furyl, the substituted aromatic heterocyclic-alkyl, and the substituted heterocyclic ring formed together with the two adjacent carbon atoms include 1 to 4 substituents which may be the same or different, such as halogen, hydroxy, nitro, azido, amino, cyano, carboxy, formyl, substituted or unsubstituted lower alkyl, substituted or unsubstituted lower alkanoyl, substituted or unsubstituted lower alkenyl, substituted or unsubstituted lower alkynyl, substituted or unsubstituted cycloalkyl, substituted or unsubstituted lower alkoxy, substituted or unsubstituted lower alkanoyloxy, substituted or unsubstituted lower alkylamino, substituted or unsubstituted di-lower alkylamino, substituted or unsubstituted lower alkylsulfanyl, substituted or unsubstituted lower alkylsulfonyl, sulfamoyl, substituted or unsubstituted lower alkylaminosulfonyl, substituted or unsubstituted di-lower alkylaminosulfonyl, substituted or unsubstituted lower alkoxycarbonyl, substituted or unsubstituted lower alkylaminocarbonyl, substituted or unsubstituted di-lower alkylaminocarbonyl, heterocyclic-carbonyl, substituted or unsubstituted aryl, a substituted or unsubstituted alicyclic heterocyclic group, a substituted or unsubstituted aromatic heterocyclic group, substituted or unsubstituted aryloxy, substituted or unsubstituted heterocyclic-oxy and tri(lower alkyl)silyl.

In the examples of the substituents (D), examples of the substituents in the substituted lower alkyl, the substituted lower alkenyl, the substituted lower alkynyl, the substituted lower alkanoyl, the substituted lower alkoxy, the substituted lower alkanoyloxy, the substituted lower alkylamino, the substituted di-lower alkylamino, the substituted lower alkylsulfanyl, the substituted lower alkylsulfonyl, the substituted lower alkylaminosulfonyl, the substituted di-lower alkylaminosulfonyl, the substituted lower alkoxycarbonyl, the substituted lower alkylaminocarbonyl and the substituted di-lower alkylaminocarbonyl include 1 to 3 substituents which may be the same or different, such as the groups described in the examples of the substituents (a).
In the examples of the substituents (D), examples of the substituents in the substituted aryl, the substituted

aryloxy, the substituted aromatic heterocyclic group and the substituted heterocyclic-oxy include 1 to 3 substituents which may be the same or different, such as the groups described in the examples of the substituents (g).
In the examples of the substituents (D), examples of the substituents (k) in the substituted cycloalkyl and the substituted alicyclic heterocyclic group include 1 to 3 substituents which may be the same or different, such as the groups described in the examples of the substituents (a), lower alkyl and oxo.

In the examples of the substituents (D) and the substituents (k), the lower alkyl moiety of the lower alkyl, the lower alkanoyl, the lower alkoxy, the lower alkanoyloxy, the lower alkylamino, the di-lower alkylamino, the lower alkylsulfanyl, the lower alkylsulfonyl, the lower alkylaminosulfonyl, the di-lower alkylaminosulfonyl, the lower alkoxycarbonyl, the lower alkylaminocarbonyl, the di-lower alkylaminocarbonyl and the tri(lower alkyl)silyl; the lower alkenyl; the lower alkynyl; the cycloalkyl; the aryl moiety of the aryl and the aryloxy; the alicyclic heterocyclic group; the aromatic heterocyclic group and the halogen have the same meanings as the lower alkyl (i), the lower alkenyl (ii), the lower alkynyl (iii), the cycloalkyl (iv), the aryl (v), the alicyclic heterocyclic group (viii), the aromatic heterocyclic group (vi) and the halogen (x) defined above, respectively; examples of the heterocyclic moiety of the heterocyclic-carbonyl and the heterocyclic-oxy include the groups described in the examples of the above-mentioned alicyclic heterocyclic group (viii), the aromatic heterocyclic group (vi), and the like; the two lower alkyl moieties of the di-lower alkylamino, the di-lower alkylaminocarbonyl and the di-lower alkylaminosulfonyl may be the same or different; and the three lower alkyl moieties of the tri(lower alkyl) silyl may be the same or different.

(xviii) Examples of the substituents (E) in the substituted alicyclic heterocyclic group, the substituted tetrahydropyranyl, the substituted dioxepanyl, the substituted alicyclic heterocyclic-alkyl and the substituted alicyclic heterocyclic group formed together with the adjacent nitrogen atom include 1 to 3 substituents which may be the same or different, such as the groups described in the examples of the substituents (D) and oxo.

Examples of the pharmaceutically acceptable salts of Compounds (I) and (IA) include pharmaceutically acceptable acid addition salts, metal salts, ammonium salts, organic amine addition salts, amino acid addition salts, and the like. Examples of the pharmaceutically acceptable acid addition salts of Compounds (I) and (IA) include inorganic acid salts such as hydrochloride, sulfate and phosphate, and organic acid salts such as acetate, maleate, fumarate, and citrate. Examples of the pharmaceutically acceptable metal salts include alkali metal salts such as sodium salts and potassium salts, alkaline earth metal salts such as magnesium salts and calcium salts, aluminum salts, zinc salts, and the like. Examples of the pharmaceutically acceptable ammonium salts include salts of ammonium, tetramethylammonium, and the like. Examples of the pharmaceutically acceptable organic amine addition salts include addition salts of morpholine, piperidine, and the like. Examples of the pharmaceutically acceptable amino acid addition salts include addition salts of lysine, glycine, phenylalanine, aspartic acid, glutamic acid, and the like.

Examples of the diseases associated with adenosine $A_{2A}$ receptor, which can be treated and/or prevented by the adenosine $A_{2A}$ receptor antagonist of the present invention, include central nervous system diseases such as Parkinson's disease, Alzheimer's disease, progressive supranuclear palsy, AIDS encephalopathy, transmissible spongiform encephalopathy, multiple sclerosis, amyotrophic lateral sclerosis, Huntington's disease, multiple system atrophy, cerebral ischemia, attention deficit hyperactivity disorder, sleep disorder, intermittent claudication, diabetes, anxiety disorders (such as panic attack and panic disorder, phobia, obsessive-compulsive disorder, posttraumatic stress disorder, acute stress disorder, generalized anxiety disorder, and anxiety caused by physical disorder or a substance), mood disorders (such as depression, dysthymic disorder, and mood-circulatory disorder), restless legs syndrome (RLS), drug dependence (such as alcohol dependence), eating disorder, epilepsy, migraine and chronic musculoskeletal pain, ischemic heart diseases such as myocardial infarction, cerebral infarction, and the like.

The processes for producing Compounds (I) are described below.
In the following production processes, when the defined groups undergo changes under the reaction conditions of the production processes or are not suitable to carry out the processes, desired compounds can be produced by methods conventionally used in synthetic organic chemistry, such as introduction and removal of protecting groups [e.g. Protective Groups in Organic Synthesis, by T.W. Greene, John Wiley & Sons Inc. (1999)]. If necessary, the order of reaction steps such as introduction of a substituent may be changed.

Compounds (I) can be produced according to the following processes.
Production Method 1
Among Compounds (I), Compound (Ia) in which $R^2$ is $-COR^6$ (wherein $R^6$ has the same meaning as defined above) and $R^3$ is $-NHCOR^7$ (wherein $R^7$ has the same meaning as defined above) can be produced according to the following steps.

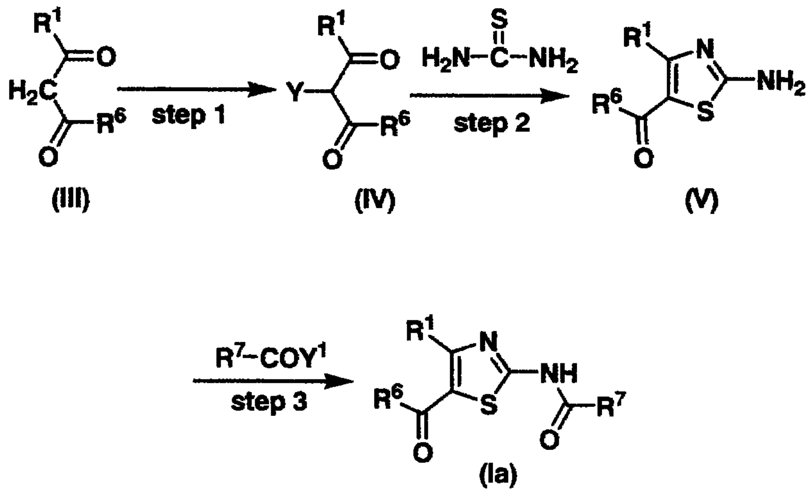


(III) (IV) (V) (Ia)

(wherein $R^1$, $R^6$ and $R^7$ have the same meanings as defined above, respectively, and Y and $Y^1$ may be the same or different, and each represents a chlorine atom, a bromine atom or an iodine atom).

Step 1

Compound (IV) can be produced by reacting Compound (III) with 1 to 200 equivalents, preferably 1 to 5 equivalents of a halogenating agent in the absence of a solvent or in a solvent inert to the reaction at a temperature between -30˚C and 150˚C, preferably at a temperature between 0˚C and 100˚C, for 5 minutes to 48 hours.

Compound (III) is commercially available or can be obtained, for example, according to the method described in WO03/35639, Japanese Published Unexamined Patent Application No. 193281/1999 or the like, or a method similar thereto.

Examples of the halogenating agent include chlorine, bromine, iodine, N,N,N,N-tetra-n-butyl ammonium tribromide, pyridinium tribromide, and the like.

Examples of the solvent inert to the reaction include acetone, 1,4-dioxane, acetonitrile, chloroform, dichloromethane, tetrahydrofuran (THF), ethyl acetate, N,N-dimethylformamide (DMF), acetic acid, water, and the like. These can be used herein either singly or in a combination.

Step 2

Compound (V) can be produced by reacting Compound (IV) with 1 to 20 equivalents of thiourea in a solvent inert to the reaction at a temperature between -30˚C and 150˚C, preferably at a temperature between room temperature and 100˚C, for 5 minutes to 48 hours.

Examples of the solvent inert to the reaction include toluene, hexane, THF, DMF, ethanol, acetonitrile, and the like. These can be used herein either singly or in a combination.

Step 3

Compound (Ia) can be produced by reacting Compound (V) with 1 to 100 equivalents of $R^7COY^1$ (wherein $R^7$ and $Y^1$ have the same meanings as defined above, respectively), in the absence of a solvent or in a solvent inert to the reaction and, if necessary, in the presence of 1 to 100 equivalents of a base at a temperature between -30˚C and 150˚C for 5 minutes to 48 hours.

Examples of the solvent inert to the reaction include acetonitrile, dichloromethane, 1,2-dichloroethane, chloroform, 1,2-dimethoxyethane, DMF, N,N-dimethylacetamide (DMA), 1,4-dioxane, THF, diethyl ether, diisopropyl ether, benzene, toluene, xylene, pyridine, N,N-dimethylimidazolidinone (DMI), N-methylpyrrolidone (NMP), sulforane, and the like. These can be used herein either singly or in a combination.

Examples of the base include pyridine, triethylamine, diisopropylethylamine, N-methylmorpholine, N-methylpiperidine, 1,8-diazabicyclo[5.4.0]undec-7-ene (DBU), N;N-dimethylaminopyridine (DMAP), potassium acetate, potassium carbonate, cesium carbonate, sodium carbonate, sodium hydrogen carbonate, sodium hydroxide, lithium hydroxide, potassium hydroxide, potassium phosphate, and the like. These can be used herein either singly or in a combination of two or more.

Production Method 2

Compound (Ia) can also be produced according to the following steps.

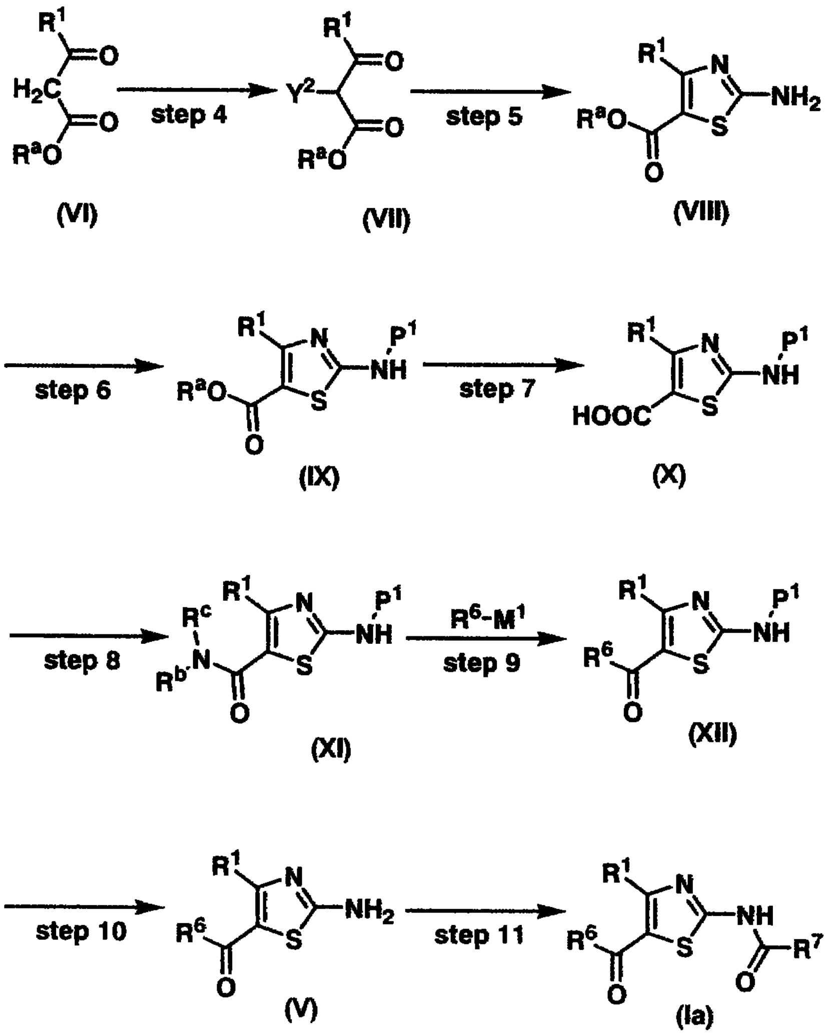


[wherein $R^1$, $R^6$ and $R^7$ have the same meanings as defined above, respectively, $Y^2$ has the same meaning as Y defined above, $R^a$ represents lower alkyl (the lower alkyl has the same meaning as defined above), $R^b$ and $R^c$ may be the same or different, and each represents lower alkyl or lower alkoxy (the lower alkyl and the lower alkoxy have the same meanings as defined above, respectively), $P^1$ represents a protecting group (examples of the protecting group include tert-butoxycarbonyl (a Boc group), benzyloxycarbonyl (a Z group), benzyl, acetyl, benzoyl, and the like), $M^1$ represents a metal group such as MgCl, MgBr, MgI, Li, $ZnR^d$ (wherein $R^d$ represents lower alkyl and the lower alkyl has the same meaning as defined above), or $Ti(OCH(CH_3)_2)_3$.]

Step 4

Compound (VII) can be produced in the same manner as in Step 1 of Production Method 1 by using Compound (VI).

Compound (VI) is commercially available or can be obtained, for example, according to the method described in WO03/35639, Japanese Published Unexamined Patent Application No. 193281/1999, or the like, or a method similar thereto.

Step 5

Compound (VIII) can be produced in the same manner as in Step 2 of Production Method 1 by using Compound (VII).

Step 6

Compound (IX) can be produced in a manner similar to the method for introducing a protecting group into an amino group described in, for example, Protective Groups in Organic Synthesis, by T. W. Greene, John Wiley & Sons Inc., 1981 or the like by using Compound (VIII).

For example, among Compounds (IX), Compounds (IX-i) in which $P^1$ is a Boc group can be produced by reacting Compound (VIII) with 1 to 30 equivalents of di-tert-butyl dicarbonate in a solvent inert to the reaction and, if necessary, in the presence of 1 to 30 equivalents of a base at a temperature between -30°C and the boiling point of the solvent used for 5 minutes to 48 hours.

Examples of the solvent inert to the reaction include 1,2-dimethoxyethane, DMF, dioxane, THF, diethyl ether, diisopropyl

ether, dichloromethane, chloroform, 1,2-dichloroethane, benzene, toluene, xylene, pyridine, NMP, DMI, sulforane, water, and the like. These can be used herein either singly or in a combination.

Examples of the base include pyridine, triethylamine, diisopropylamine, DBU, DMAP, N-methylmorpholine, N-methylpiperidine, potassium acetate, potassium carbonate, cesium carbonate, sodium carbonate, sodium hydrogen carbonate, sodium hydroxide, lithium hydroxide, potassium hydroxide, potassium phosphate, and the like. These can be used herein either singly or in a combination of two or more.

Step 7

Compound (X) can be produced in a manner similar to the method for removing a protecting group described in, for example, Protective Groups in Organic Synthesis, by T. W. Greene, John Wiley & Sons Inc., 1981 or the like by using Compound (IX).

For example, when $R^a$ is methyl or ethyl, Compound (X) can be produced by treating Compound (IX) with 1 equivalent to a large excessive amount of a base in a solvent containing water at a temperature between 0˚C and the boiling point of the solvent used for 5 minutes to 48 hours.

Examples of the solvent include methanol, ethanol, propanol, THF, 1,4-dioxane, 1,2-dimethoxyethane, toluene, dichloromethane, DMF, and the like. These can be used herein either singly or in a combination.

Examples of the base include sodium hydroxide, potassium hydroxide, lithium hydroxide, and the like.

Further, for example, when $R^a$ is tert-butyl, Compound (X) can be produced by treating Compound (IX) with 1 equivalent to a large excessive amount of an acid in the absence of a solvent or in a solvent inert to the reaction at a temperature between -30˚C and 100˚C for 5 minutes to 48 hours.

Examples of the solvent inert to the reaction include methanol, ethanol, propanol, THF, 1,4-dioxane, 1,2-dimethoxyethane, toluene, ethyl acetate, dichloromethane, DMF, water, and the like. These can be used herein either singly or in a combination.

Examples of the acid include trifluoroacetic acid, hydrochloric acid, sulfuric acid, and the like.

Step 8

Compound (XI) can be produced by reacting Compound (X) with 1 to 100 equivalents of $HNR^bR^c$ (wherein $R^b$ and $R^c$ have the same meanings as defined above, respectively) in the absence of a solvent or in a solvent inert to the reaction in the presence of 1 to 30 equivalents of a condensing agent and, if necessary, in the presence of 1 to 30 equivalents of an additive at a temperature between -30˚C and 100˚C for 5 minutes to 72 hours.

Examples of the solvent inert to the reaction include acetonitrile, dichloromethane, 1,2-dichloroethane, chloroform, 1,2-dimethoxyethane, DMF, DMA, 1,4-dioxane, THF, diethyl ether, diisopropyl ether, benzene, toluene, xylene, pyridine, DMI, NMP, sulforane, water, and the like. These can be used herein either singly or in a combination.

Examples of the condensing agent include dicyclohexylcarbodiimide (DCC), diisopropylcarbodiimide, N-(3-dimethylaminopropyl)-N'-ethylcarbodiimide (EDC), EDC hydrochloride, benzotriazol-1-yl-tris (dimethylamino)phosphonium hexafluorophosphate (BOP), benzotriazol-1-yl-tripyrrolidinophosphonium hexafluorophosphate (PyBOP), diphenylphosphorylazide (DPPA), and the like.

Examples of the additive include 1-hydroxybenzotriazole hydrate, triethylamine, DMAP, and the like. These can be used herein either singly or in a combination.

Step 9

Compound (XII) can be produced by reacting Compound (XI) with 1 to 50 equivalents of $R^6M^1$ (wherein $R^6$ and $M^1$ have the same meanings as defined above, respectively) in a solvent inert to the reaction at a temperature between - 78˚C and the boiling point of the solvent used for 5 minutes to 48 hours.

Examples of the solvent inert to the reaction include diethyl ether, THF, 1,4-dioxane, 1,2-dimethoxyethane, toluene, and the like. These can be used herein either singly or in a combination.

Step 10

Compound (V) can be produced in a manner similar to the method for removing a protecting group described in, for example, Protective Groups in Organic Synthesis, by T. W. Greene, John Wiley & Sons Inc., 1981 or the like by using Compound (XII).

For example, when $P^1$ is a Boc group, Compound (V) can be produced by treating Compound (XII) with 1 equivalent to a large excessive amount of an acid in the absence of a solvent or in a solvent inert to the reaction at a temperature between -30˚C and 150˚C for 5 minutes to 48 hours.

Examples of the solvent inert to the reaction include dichloromethane, chloroform, 1,2-dichloroethane, methanol, ethanol, THF, ethyl acetate, water, and the like. These can be used herein either singly or in a combination.

Examples of the acid include trifluoroacetic acid, hydrochloric acid, sulfuric acid, and the like.

Step 11

Compound (Ia) can be produced in the same manner as in Step 3 of Production Method 1 by using Compound (V).

Production Method 3

Compound (Ia) can also be produced according to the following steps.

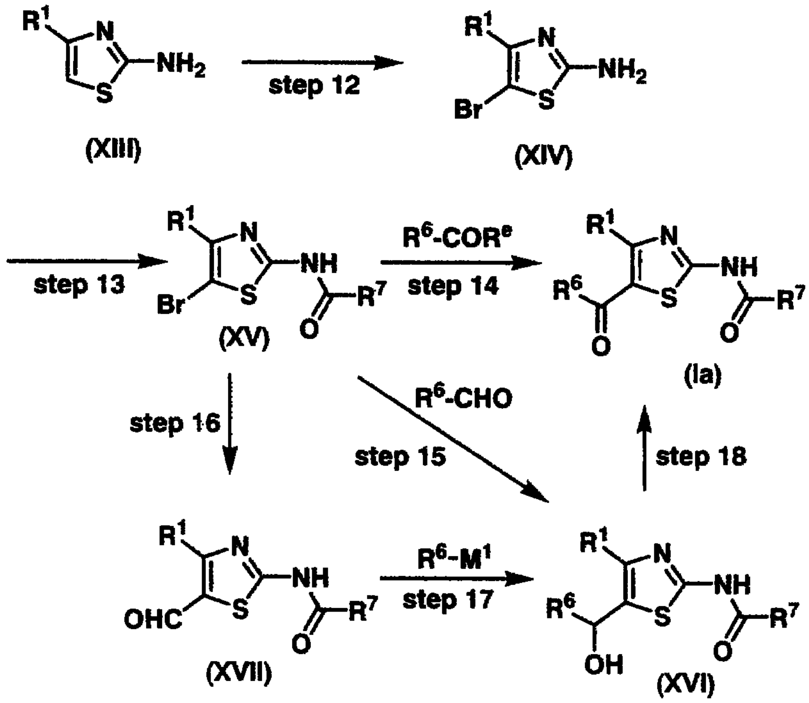


[wherein $R^1$, $R^6$, $R^7$ and $M^1$ have the same meanings as defined above, respectively, $R^e$ represents lower alkoxy, substituted or unsubstituted aryloxy (the lower alkoxy and the aryl moiety of the aryloxy have the same meanings as the lower alkoxy and the aryl defined above, respectively, examples of the substituents in the substituted aryloxy include 1 to 3 substituents which may be the same or different, such as lower alkyl, halogen and lower alkoxy, and the lower alkyl, the halogen and the lower alkoxy have the same meanings as defined above, respectively) or $-NR^bR^c$ (wherein $R^b$ and $R^c$ have the same meanings as defined above, respectively)]

Step 12

Compound (XIV) can be produced according to the method described in, for example, Journal of the Chemical Society, 1947, p. 114 or the like, or a method similar thereto by using Compound (XIII).

For example, Compound (XIV) can be produced by reacting Compound (XIII) with 1 to 20 equivalents of a brominating agent in a solvent inert to the reaction at a temperature between -30°C and the boiling point of the solvent used, for 5 minutes to 48 hours.

Compound (XIII) is commercially available or can be obtained, for example, according to the method described in Journal of the American Chemical Society, 1953, Vol. 72, p. 3722 or the like, or a method similar thereto.

Examples of the solvent inert to the reaction include dichloromethane, chloroform, 1,2-dichloroethane, and the like. These can be used herein either singly or in a combination.

Examples of the brominating agent include N-bromosuccinimide, bromine, N,N,N,N-tetra-n-butyl ammonium bromide, and the like.

Step 13

Compound (XV) can be produced in the same manner as in Step 3 of Production Method 1 by using Compound (XIV).

Step 14

Compound (Ia) can be produced by reacting Compound (XV) with 1 to 100 equivalents of $R^6$-$COR^e$ in a solvent inert to the reaction in the presence of 1 to 20 equivalents of a base at a temperature between -78°C and room temperature for 5 minutes to 48 hours.

Examples of the solvent inert to the reaction include THF, diethyl ether, 1,4-dioxane, 1,2-dimethoxyethane, hexane, and the like. These can be used herein either singly or in a combination.

Examples of the base include lithium diisopropylamide, lithium bis(trimethylsilyl)amide, methyllithium, n-butyllithium, lithium hydride, sodium hydride, potassium hydride, methylmagnesium bromide, ethylmagnesium bromide, isopropylmagnesium chloride, and the like. These can be used herein either singly or in a combination of two or more. Step 15

Compound (XVI) can be produced by reacting Compound (XV) with 1 to 100 equivalents of $R^6CHO$ in a solvent inert to the reaction in the presence of 1 to 20 equivalents of a base at a temperature between -78°C and room temperature for 5 minutes to 48 hours.

Examples of the solvent inert to the reaction include THF, diethyl ether, 1,4-dioxane, 1,2-dimethoxyethane, hexane, and the like. These can be used herein either singly or in a combination.
Examples of the base include lithium diisopropylamide, lithium bis(trimethylsilyl)amide, methyllithium, n-butyllithium, lithium hydride, sodium hydride, potassium hydride, methylmagnesium bromide, ethylmagnesium bromide, isopropylmagnesium chloride, and the like. These can be used herein either singly or in a combination of two or more.
Step 16
Compound (XVII) can be produced by reacting Compound (XV) with 1 to 100 equivalents of a formylating agent in a solvent inert to the reaction in the presence of 1 to 20 equivalents of a base at a temperature between -78°C and room temperature for 5 minutes to 48 hours.
Examples of the solvent inert to the reaction include THF, diethyl ether, 1,4-dioxane, 1,2-dimethoxyethane, hexane, and the like. These can be used herein either singly or in a combination.
Examples of the formylating agent include DMF, N-methyl-N-phenylformamide, N-methyl-N-(2-pyridyl) formamide, morpholinoformamide, and the like.
Examples of the base include lithium diisopropylamide, lithium bis(trimethylsilyl)amide, methyllithium, n-butyllithium, lithium hydride, sodium hydride, potassium hydride, methylmagnesium bromide, ethylmagnesium bromide, isopropylmagnesium chloride, and the like. These can be used herein either singly or in a combination of two or more.
Step 17
Compound (XVI) can be produced in the same manner as in Step 9 of Production Method 2 by using Compound (XVII) and $R^6M^1$ (wherein $R^6$ and $M^1$ have the same meanings as defined above, respectively).
Step 18
Compound (Ia) can be produced by treating Compound (XVI) with 1 to 100 equivalents of an oxidizing agent in the absence of a solvent or in a solvent inert to the reaction at a temperature between -78°C and the boiling point of the solvent used for 5 minutes to 48 hours.
Examples of the solvent inert to the reaction include acetonitrile, dichloromethane, 1,2-dichloroethane, chloroform, 1,2-dimethoxyethane, DMF, DMA, 1,4-dioxane, THF, diethyl ether, diisopropyl ether, benzene, toluene, xylene, pyridine, DMI, NMP, sulforane, water, and the like. These can be used herein either singly or in a combination.
Examples of the oxidizing agent include chromic acid, pyridinium chlorochromate (PCC), pyridinium dichromate (PDC), dimethylsulfoxide (DMSO)-oxalyl chloride, DMSO-dicyclohexylcarbodiimide (DCC), tetrapropylammonium perruthenate (TPAP), Dess-Martin reagent (DMP: 1,1,1-triacetoxy-1,1-dihydro-1,2-benziodoxol-3(1H)-one, Lecture of Experimental Chemistry, 5th Ed., Vol. 15, p. 27, by the Chemical Society of Japan, Maruzen, 2003), 2-iodoxylbenzoic acid (IBX), 2,2,6,6-tetramethylpiperidine-N-oxide (TEMPO), manganese dioxide, and the like.
Production Method 4
Compound (Ia) can also be produced according to the following steps.
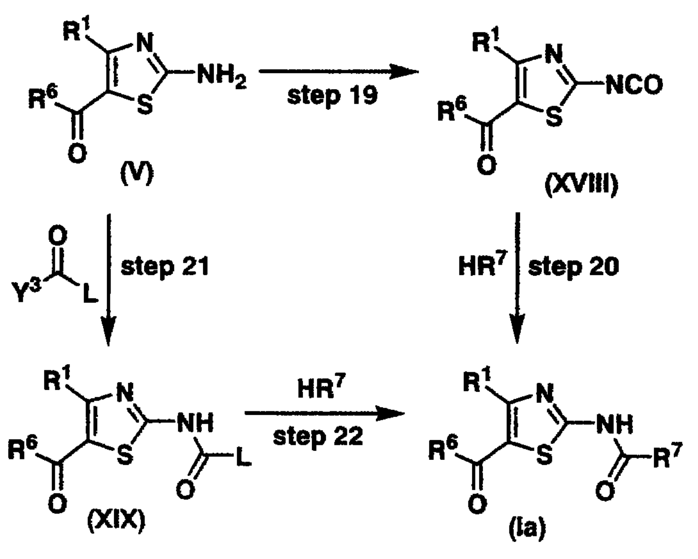


[wherein, $R^1$, $R^6$ and $R^7$ have the same meanings as defined above, respectively, $Y^3$ has the same meaning as Y defined above, L represents halogen, lower alkoxy or substituted or unsubstituted aryloxy (the halogen, the lower alkoxy and the aryloxy have the same meanings as defined above, respectively, examples of the substituents in the

substituted aryloxy include 1 to 3 substituents which may be the same or different, such as lower alkyl, halogen and lower alkoxy, and the lower alkyl, the halogen and the lower alkoxy have the same meanings as defined above, respectively)]

Step 19

Compound (XVIII) can be produced according to the method described in Lecture of Experimental Chemistry, 4th Ed., Vol. 20, pp. 473-483, by the Chemical Society of Japan, Maruzen, 1992, or a method similar thereto by using Compound (V).

For example, Compound (XVIII) can be produced by reacting Compound (V) with 1 to 20 equivalents of phosgene or a phosgene equivalent in the absence of a solvent or in a solvent inert to the reaction and, if necessary, in the presence of 1 to 100 equivalents of a base at a temperature between -30°C and 150°C for 5 minutes to 72 hours. Examples of the solvent inert to the reaction include acetonitrile, methanol, ethanol, dichloromethane, chloroform, 1,2-dichloroethane, 1,2-dimethoxyethane, DMF, DMA, 1,4-dioxane, THF, diethyl ether, diisopropyl ether, benzene, toluene, xylene, DMI, NMP, sulforane, water, and the like. These can be used herein either singly or in a combination.

Examples of the phosgene equivalent include triphosgene, 1,1'-carbonyldiimidazole (CDI), and the like. Examples of the base include pyridine, triethylamine, diisopropylethylamine, N-methylmorpholine, N-methylpiperidine, DBU, DMAP, potassium acetate, potassium carbonate, cesium carbonate, sodium carbonate, sodium hydrogen carbonate, sodium hydroxide, lithium hydroxide, potassium hydroxide, potassium phosphate, and the like. These can be used herein either singly or in a combination of two or more.

Step 20

Compound (Ia) can be produced by reacting Compound (XVIII) with 1 to 200 equivalents of $HR^7$ (wherein $R^7$ has the same meaning as defined above) in the absence of a solvent or in a solvent inert to the reaction and, if necessary, in the presence of 1 to 100 equivalents of a base at a temperature between -30°C and 150°C for 5 minutes to 72 hours. Examples of the solvent inert to the reaction include acetonitrile, dichloromethane, chloroform, 1,2-dichloroethane, 1,2-dimethoxyethane, DMF, DMA, 1,4-dioxane, THF, diethyl ether, diisopropyl ether, benzene, toluene, xylene, DMI, NMP, sulforane, water, and the like. These can be used herein either singly or in a combination. Among these, DMF or DMA is preferred.

Examples of the base include triethylamine, diisopropylethylamine, DBU, and the like.

Further, Compound (Ia) can also be produced by continuously performing Step 20 without isolating Compound (XVIII) in Step 19.

Step 21

Compound (XIX) can be produced by reacting Compound (V) with 1 to 50 equivalents of $Y^3COL$ (wherein $Y^3$ and L have the same meanings as defined above, respectively) in a solvent inert to the reaction and, if necessary, in the presence of 1 to 100 equivalents of a base at a temperature between -30°C and 150°C for 5 minutes to 48 hours.

$Y^3COL$ is commercially available or can be obtained, for example, according to the method described in Journal of the American Chemical Society, 1954, Vol. 76, p. 4458 or the like, or a method similar thereto.

Examples of the solvent inert to the reaction include acetonitrile, dichloromethane, 1,2-dichloroethane, chloroform, 1,2-dimethoxyethane, DMF, DMA, 1,4-dioxane, THF, diethyl ether, diisopropyl ether, benzene, toluene, xylene, pyridine, DMI, NMP, sulforane, and the like. These can be used herein either singly or in a combination.

Examples of the base include pyridine, triethylamine, diisopropylethylamine, N-methylmorpholine, N-methylpiperidine, DBU, DMAP, potassium acetate, potassium carbonate, cesium carbonate, sodium carbonate, sodium hydrogen carbonate, sodium hydroxide, lithium hydroxide, potassium hydroxide, potassium phosphate, and the like. These can be used herein either singly or in a combination of two or more.

Step 22

Compound (Ia) can be produced in the same manner as in Step 20 of Production Method 4 by using Compound (XIX).

Production Method 5

Among Compounds (I), Compound (Ib) in which $R^3$ is $-NHCONHR^8$ (wherein $R^8$ has the same meaning as defined above) can also be produced according to the following steps.

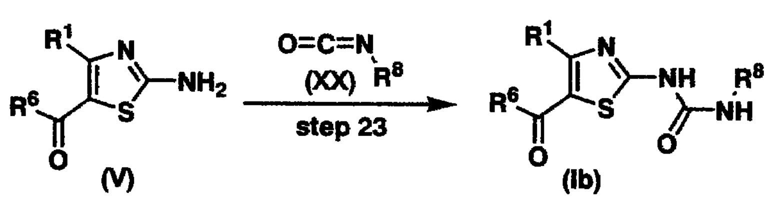

(wherein $R^1$, $R^6$ and $R^8$ have the same meanings as defined above, respectively.)
Step 23
Compound (Ib) can be produced by reacting Compound (V) with 1 to 20 equivalents of Compound (XX) in a solvent inert to the reaction at a temperature between 0°C and the boiling point of the solvent used for 5 minutes to 48 hours.
Compound (XX) is commercially available or can be obtained according to the method described in Lecture of Experimental Chemistry, 4th Ed., Vol. 20, p. 473-483, by the Chemical Society of Japan, Maruzen, 1992 or the like, or a method similar thereto.
Examples of the solvent inert to the reaction include acetonitrile, THF, 1,4-dioxane, DMF, dichloromethane, 1,2-dichloroethane, diethyl ether, benzene, toluene, water, and the like.
Production Method 6
Among Compounds (I), Compound (Ic) in which $R^3$ is - $CONHR^{11}$ (wherein $R^{11}$ has the same meaning as defined above) can be produced according to the following steps.
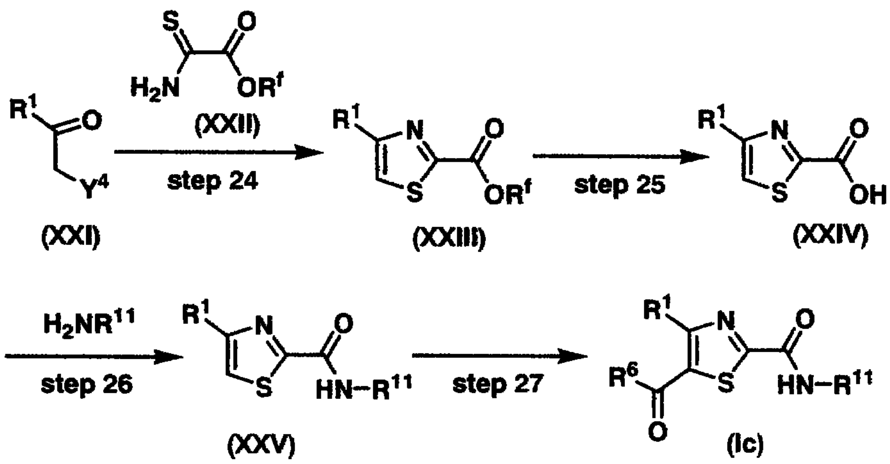


(wherein $R^1$, $R^6$ and $R^{11}$ have the same meanings as defined above, respectively, and $R^f$ and $Y^4$ have the same meanings as $R^a$ and Y defined above, respectively)
Step 24
Compound (XXIII) can be produced according to the method described in Journal of Medicinal and Pharmaceutical Chemistry, 1959, Vol. 2, p. 588, or a method similar thereto by using Compound (XXI).
For example, Compound (XXIII) can be produced by reacting Compound (XXI) with 1 to 20 equivalents of Compound (XXII) in a solvent inert to the reaction at a temperature between room temperature and 100°C for 5 minutes to 48 hours.
Examples of the solvent inert to the reaction include toluene, hexane, THF, DMF, methanol, ethanol, diethyl ether, acetonitrile, and the like. These can be used herein either singly or in a combination.
Compound (XXII) is commercially available or can be obtained according to the method described in Journal of Medicinal and Pharmaceutical Chemistry, 1959, Vol. 2, p. 588 or the like, or a method similar thereto.
Compound (XXI) can be obtained in the same manner as in Step 1 of Production Method 1 by using $R^1COCH_3$ (wherein $R^1$ has the same meaning as defined above).
$R^1COCH_3$ is commercially available or can be obtained, for example, according to the method described in WO03/35639, or Japanese Published Unexamined Patent Application No. 193281/1999, or a method similar thereto.
Further, Compound (XXIII) can also be produced by performing Step 24 as such without isolating Compound (XXI).
Step 25
Compound (XXIV) can be produced in the same manner as in Step 7 of Production Method 2 by using Compound (XXIII).
Step 26
Compound (XXV) can be produced in the same manner as in Step 8 of Production Method 2 by using Compound (XXIV) and $H_2NR^{11}$ (wherein $R^{11}$ has the same meaning as defined above).
Step 27
Compound (Ic) can be produced in the same manner as in Step 14 of Production Method 3 by using Compound (XXV).
Production Method 7
Compound (Ic) can also be produced according to the following steps.
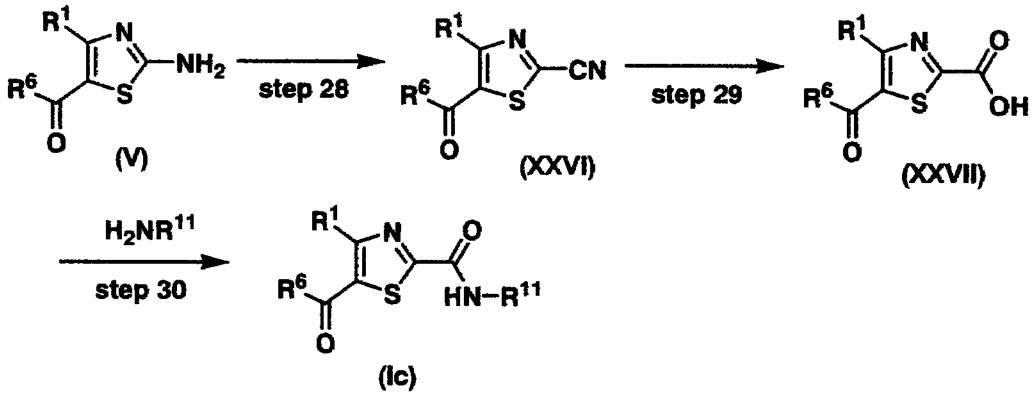


(V) (XXVI) (XXVII) (Ic)

Step 28
Compound (XXVI) can be produced by reacting Compound (V) with 1 to 20 equivalents of a cyaniding agent in a solvent inert to the reaction in the presence of 1 to 20 equivalents of a nitrite compound at a temperature between -30˚C and 150˚C for 5 minutes to 48 hours.
Examples of the nitrite compound include sodium nitrite, tert-butyl nitrite, isoamyl nitrite, and the like.
Examples of the cyaniding agent include copper(I) cyanide, sodium cyanide, potassium cyanide, tetrabutylammonium cyanide, zinc cyanide, potassium copper(I) cyanide, and the like.
Examples of the solvent inert to the reaction include toluene, acetonitrile, dimethylsulfoxide, water, and the like. These can be used herein either singly or in a combination.
Step 29
Compound (XXVII) can be produced according to the method described in Lecture of Experimental Chemistry, 5th Ed., Vol. 15, p. 15, by the Chemical Society of Japan, Maruzen, 2003, or a method similar thereto by using Compound (XXVI).
For example, Compound (XXVII) can be produced by treating Compound (XXVI) with 1 equivalent to a large excessive amount of a base in a solvent containing water at a temperature between 0˚C and the boiling point of the solvent used for 5 minutes to 48 hours.
Examples of the base include sodium hydroxide, potassium hydroxide, lithium hydroxide, and the like.
Examples of the solvent include methanol, ethanol, propanol, THF, 1,4-dioxane, 1,2-dimethoxyethane, toluene, dichloromethane, DMF, and the like. These can be used herein either singly or in a combination.
Step 30
Compound (Ic) can be produced in the same manner as in Step 8 of Production Method 2 by using Compound (XXVII) and $H_2NR^{11}$ (wherein $R^{11}$ has the same meaning as defined above).
Production Method 8
Among Compounds (I), Compound (Id) in which $R^3$ is the above-mentioned general formula (II) can be produced according to the following steps.
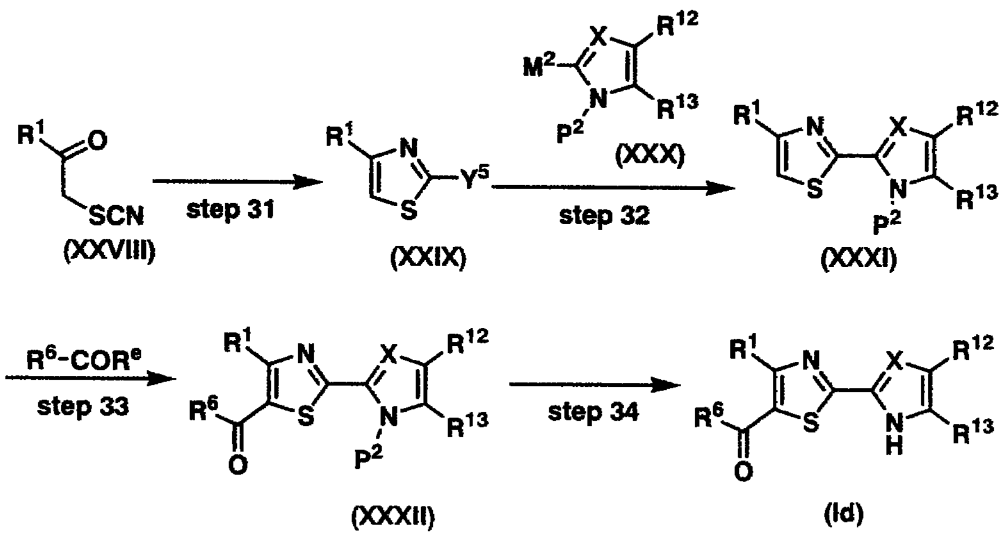


[wherein $R^1$, $R^6$, $R^{12}$, $R^{13}$, $R^e$ and X have the same meanings as defined above, respectively, $Y^5$ has the same meaning as Y defined above, $P^2$ represents a hydrogen atom or a protecting group (examples of the protecting group include tert-butoxycarbonyl (a Boc group), benzyloxycarbonyl (a Z group), benzyl, acetyl, benzoyl, and the like), $M^2$ represents trimethyltin, tributyltin, triphenyltin, -B($OR^9$)$OR^h$ (wherein $R^9$ and $R^h$ may be the same or different, and each represents a hydrogen atom or lower alkyl, and the lower alkyl has the same meaning as defined above) or 4,4,5,5-tetramethyl-1,3,2-dioxaborolane]

Step 31

Compound (XXIX) can be produced by reacting Compound (XXVIII) with 1 equivalent to a large excessive amount of a hydrogen halide in a solvent inert to the reaction at a temperature between -30°C and the boiling point of the solvent used for 5 minutes to 48 hours.

Compound (XXVIII) can be obtained, for example, according to the method described in Journal of Organic Chemistry, 1948, Vol. 13, p. 722 or the like, or a method similar thereto.

Examples of the hydrogen halide include hydrogen chloride, hydrogen bromide, hydrogen iodide, and the like.

Examples of the solvent inert to the reaction include dichloromethane, chloroform, 1,2-dichloroethane, 1,4-dioxane, THF, diethyl ether, diisopropyl ether, acetonitrile, acetic acid, ethyl acetate, and the like. These can be used herein either singly or in a combination. Step 32

Compound (XXXI) can be produced by reacting Compound (XXIX) with Compound (XXX) in a solvent inert to the reaction in the presence of a catalytic amount of a palladium compound at a temperature between room temperature and 140°C for 5 minutes to 48 hours.

Compound (XXX) can be obtained according to the method described in Journal of Organic Chemistry, 2002, Vol. 67, p. 7551, Synlett, 1992, p. 805, WO03/053925 or the like, or a method similar thereto.

The reaction can also be carried out by adding 0.2 to 5 equivalents of an inorganic salt such as lithium chloride, potassium chloride, silver oxide, copper oxide, silver nitrate or silver acetate, or a base such as triethylamine, sodium ethoxide, sodium carbonate or sodium hydroxide. Among the bases, sodium carbonate is preferred.

Examples of the palladium compound include bis(triphenylphosphine)palladium(II) dichloride, tetrakis (triphenylphosphine)palladium(0), [1,2-bis(diphenyl phosphino)ethane]palladium(II) dichloride, [1,1'-bis (diphenylphosphino) ferrocene]palladium(II) dichloride, and the like.

Examples of the solvent inert to the reaction include diethyl ether, THF, 1,4-dioxane, DMF, DMA, DMSO, benzene, toluene, xylene, dichloromethane, chloroform, carbon tetrachloride, 1,2-dichloroethane, acetonitrile, ethyl acetate, methyl acetate, methyl ethyl ketone, methanol, ethanol, propanol, isopropyl alcohol, butanol, hexane, water, and the like. These can be used herein either singly or in a combination.

Step 33

Compound (XXXII) can be produced in the same manner as in Step 14 of Production Method 3 by using Compound (XXXI).

Step 34

Compound (Id) can be produced in the same manner as in Step 10 of Production Method 2 by using Compound (XXXII).

Production Method 9

Among Compounds (Id), Compound (Id-i) in which =X-is =N-, and $R^{12}$ and $R^{13}$ are $R^{12a}$ and $R^{13a}$ (wherein $R^{12a}$ and $R^{13a}$ may be the same or different, and each represents a hydrogen atom, halogen, hydroxy, nitro, azido, amino, cyano, carboxy, formyl, substituted or unsubstituted lower alkyl, substituted or unsubstituted lower alkenyl, substituted or unsubstituted lower alkynyl, substituted or unsubstituted lower alkanoyl, substituted or unsubstituted lower alkoxy, substituted or unsubstituted cycloalkyl, substituted or unsubstituted lower alkylamino, substituted or unsubstituted di-lower alkylamino, substituted or unsubstituted aryl, substituted or unsubstituted aralkyl, substituted or unsubstituted aroyl, a substituted or unsubstituted alicyclic heterocyclic group, a substituted or unsubstituted aromatic heterocyclic group, substituted or unsubstituted alicyclic heterocyclic-alkyl, or substituted or unsubstituted aromatic heterocyclic-alkyl in the definitions of $R^{12}$ and $R^{13}$), respectively can also be produced according to the following steps.

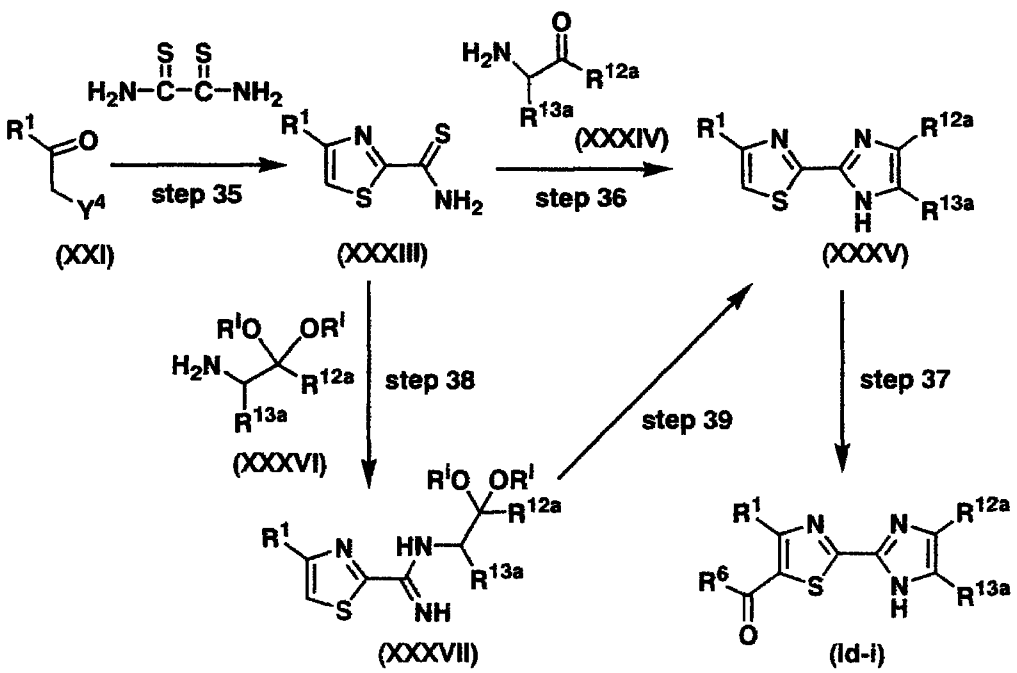


(XXI) (XXXIII) (XXXIV) (XXXV) (XXXVI) (XXXVII) (Id-i)

(wherein $R^1$, $R^6$, $R^{12a}$, $R^{13a}$ and $Y^4$ have the same meanings as defined above, respectively, and $R^i$ has the same meaning as $R^a$ defined above)

Step 35

Compound (XXXIII) can be produced according to the method described in Synthetic Communications, 2001, Vol. 31, p. 3747 or the like, or a method similar thereto.

For example, Compound (XXXIII) can be produced by reacting Compound (XXI) with 1 to 20 equivalents of rubeanic acid in a solvent inert to the reaction at a temperature between room temperature and 100˚C for 5 minutes to 48 hours.

Examples of the solvent inert to the reaction include toluene, hexane, THF, DMF, methanol, ethanol, diethyl ether, acetonitrile and the like. These can be used herein either singly or in a combination.

Step 36

Compound (XXXV) can be produced by reacting Compound (XXXIII) with a large excessive amount of methyl iodide in a solvent inert to the reaction at a temperature between 0˚C and 40˚C for 5 minutes to 72 hours followed by reacting the resulting product with Compound (XXXIV) at a temperature between room temperature and the boiling point of the solvent used in the reaction for 5 minutes to 48 hours.

Compound (XXXIV) is commercially available or can be obtained according to the method described in Journal of the Chemical Society, 1949, Vol. 71, p. 2473, Synthetic Communications, 2000, Vol. 30, p. 2287 or the like, or a method similar thereto.

Examples of the solvent inert to the reaction include toluene, hexane, THF, DMF, methanol, ethanol, diethyl ether, acetonitrile and the like. These can be used herein either singly or in a combination.

Step 37

Compound (Id-i) can be produced in the same manner as in Step 14 of Production Method 3 by using Compound (XXXV).

Step 38

Compound (XXXVII) can be produced in the same manner as in Step 36 of Production Method 9 by using Compound (XXXIII) and Compound (XXXVI).

Compound (XXXVI) is commercially available or can be obtained according to the method described in Tetrahedron, 1995, Vol. 51, p. 5147, or a method similar thereto.
Further, the compound may be directly converted into Compound (XXXV) under the reaction conditions of this step in some cases.
Step 39
Compound (XXXV) can be produced in a manner similar to the method for removing a protecting group described in, for example, Protective Groups in Organic Synthesis, by T. W. Greene, John Wiley & Sons Inc., 1981 or the like by using Compound (XXXVII).

For example, Compound (XXXV) can be produced by treating Compound (XXXVII) with 1 equivalent to a large excessive amount of an acid in a solvent inert to the reaction at a temperature between 0°C and the boiling point of the solvent used for 5 minutes to 48 hours.
Examples of the acid include hydrochloric acid, sulfuric acid, acetic acid, trifluoroacetic acid and the like.
Examples of the solvent inert to the reaction include dichloromethane, chloroform, acetone, diethyl ether, THF, water and the like. These can be used herein either singly or in a combination.
Production Method 10
Compound (Id-i) can also be produced according to the following steps.

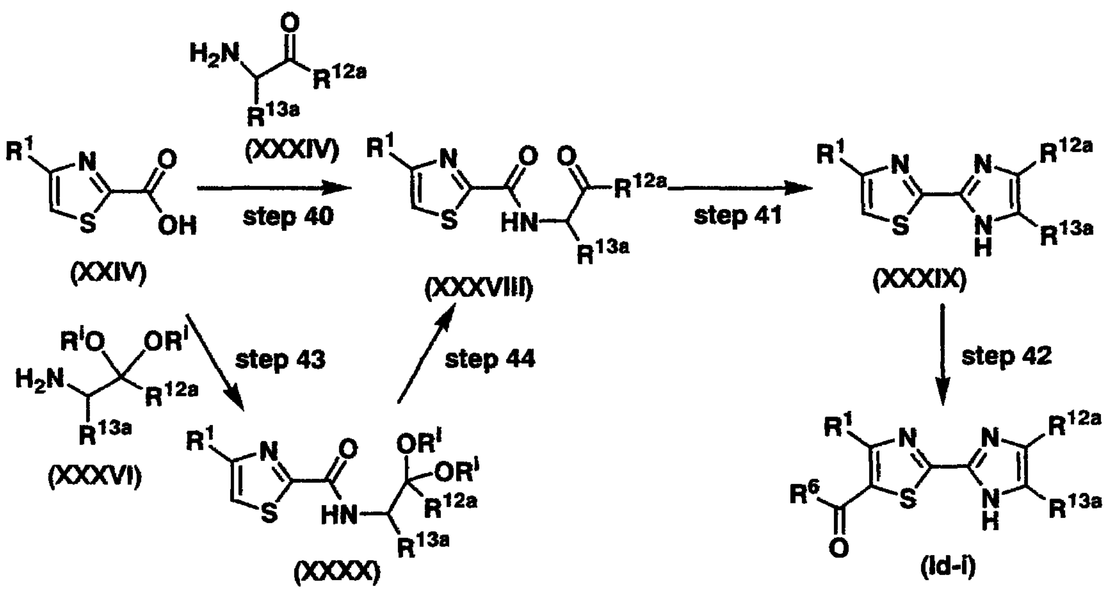


(wherein $R^1$, $R^6$, $R^{12a}$, $R^{13a}$ and $R^i$ have the same meanings as defined above, respectively)
Step 40
Compound (XXXVIII) can be produced in the same manner as in Step 8 of Production Method 2 by using Compound (XXIV) and Compound (XXXIV).
Step 41
Compound (XXXIX) can be produced by reacting Compound (XXXVIII) with 1 equivalent to a large excessive amount of an ammonia compound in a solvent inert to the reaction or in the absence of a solvent at a temperature between room temperature and 150°C.

Examples of the ammonia compound include ammonia, ammonium formate, ammonium acetate, ammonium trifluoroacetate and the like.
Examples of the solvent inert to the reaction include acetic acid, DMF and the like. These can be used herein either singly or in a combination.
Step 42
Compound (Id-i) can be produced in the same manner as in Step 14 of Production Method 3 by using Compound (XXXIX).
Step 43
Compound (XXXX) can be produced in the same manner as in Step 8 of Production Method 2 by using Compound (XXIV) and Compound (XXXVI).
Step 44
Compound (XXXVIII) can be produced in the same manner as in Step 39 of Production Method 9 by using Compound (XXXX).

Production Method 11
Among Compounds (Id), Compound (Id-ii) in which $R^{12}$ and $R^{13}$ are $R^{12a}$ and $R^{13a}$ (wherein each of $R^{12b}$ and $R^{13b}$ represents a substituted or unsubstituted carbon ring, or a substituted or unsubstituted heterocyclic ring to be formed by combining each of $R^{12}$ and $R^{13}$ with two carbon atoms adjacent thereto in the definitions of $R^{12}$ and $R^{13}$), respectively can also be produced according to the following steps.

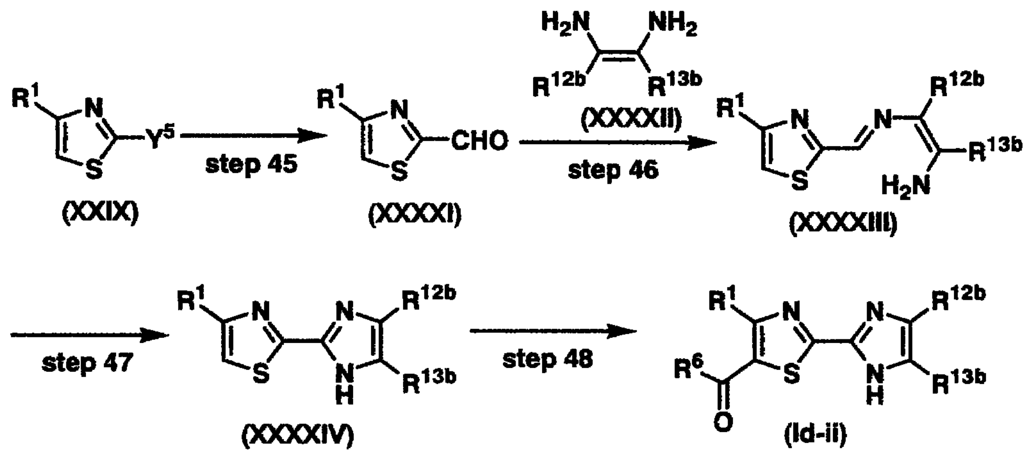

(XXIX) (XXXXI) (XXXXII) (XXXXIII) (XXXXIV) (Id-ii)

(wherein $R^1$, $R^6$, $R^{12b}$ and $R^{13b}$ have the same meanings as defined above, respectively, and $Y^5$ has the same meaning as Y defined above)

Step 45
Compound (XXXXI) can be produced in the same manner as in Step 16 of Production Method 3 by using Compound (XXIX).

Step 46
Compound (XXXXIII) can be produced by reacting Compound (XXXXI) with 1 to 200 equivalents of Compound (XXXXII) in a solvent at a temperature between -30˚C and the boiling point of the solvent used in the reaction for 5 minutes to 48 hours.

Compound (XXXXII) is commercially available or can be obtained according to the method described in Journal of Chemical Society, 1957, p. 2197, Journal of Organic Chemistry, 1960, Vol. 25, p. 1752, or a method similar thereto. Examples of the solvent include methanol, ethanol, dichloromethane, chloroform, 1,2-dichloroethane, THF, 1,4-dioxane, diethyl ether, diisopropyl ether, benzene, nitrobenzene, toluene, xylene, DMF, water, and the like. These can be used herein either singly or in a combination.

Further, in this step, when nitrobenzene is used as the solvent, Compound (XXXXIV), which is a desired compound of the subsequent step of Step 47, can be directly produced from Compound (XXXXI).

Step 47
Compound (XXXXIV) can be produced by treating Compound (XXXXIII) with 1 to 100 equivalents of an oxidizing agent in a solvent inert to the reaction at a temperature between -78˚C and the boiling point of the solvent used in the reaction for 5 minutes to 48 hours.

Examples of the oxidizing agent include m-chloroperbenzoic acid, 2,3-dichloro-5,6-dicyano-1,4-benzoquinone (DDQ), benzofuroxan, potassium permanganate, hydrogen peroxide, peracetic acid, tert-butylhydroperoxide and the like. Examples of the solvent inert to the reaction include acetonitrile, dichloromethane, 1,2-dichloroethane, chloroform, 1,2-dimethoxyethane, DMF, DMA, 1,4-dioxane, THF, diethyl ether, diisopropyl ether, benzene, toluene, xylene, pyridine, DMI, NMP, sulforane, water and the like. These can be used herein either singly or in a combination. Step 48
Compound (Id-ii) can be produced in the same manner as in Step 14 of Production Method 3 by using Compound (XXXXIV).

Further, Compounds (I) having a desired functional group at a desired position can be obtained by performing the above-mentioned processes in an appropriate combination.
For example,

Production Method 12
Compound (Id) can also be produced according to the following steps.

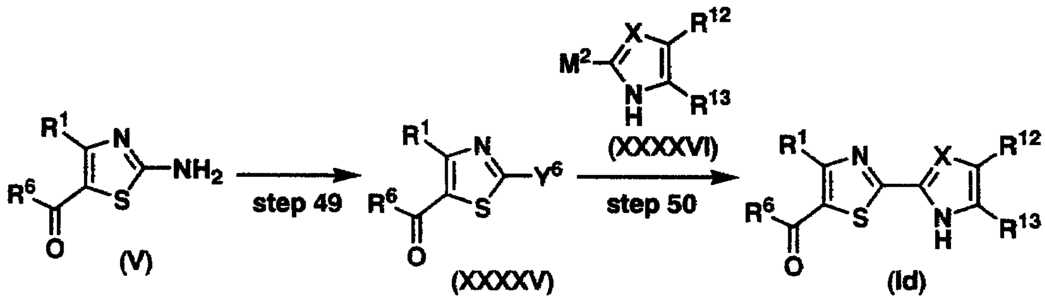


(wherein X, $R^1$, $R^6$, $R^{12}$, $R^{13}$ and $M^2$ have the same meanings as defined above, respectively, and $Y^6$ has the same meaning as Y defined above)

Step 49

Compound (XXXXV) can be produced by reacting Compound (V) with 1 to 20 equivalents of a copper(II) halide in a solvent inert to the reaction in the presence of 1 to 20 equivalents of a nitrite compound at a temperature between -30˚C and 150˚C for 5 minutes to 48 hours.

Examples of the nitrite compound include sodium nitrite, tert-butyl nitrite, isoamyl nitrite and the like.

Examples of the copper(II) halide include copper(II) bromide, copper(II) iodide and the like.

Examples of the solvent inert to the reaction include toluene, acetonitrile, dimethylsulfoxide, water and the like. These can be used herein either singly or in a combination.

Step 50

Compound (Id) can be produced in the same manner as in Step 32 of Production Method 8 by using Compound (XXXXV).

The intermediates and the desired compounds in the above-mentioned respective production processes can be isolated and purified by being subjected to a separation and purification method conventionally used in organic synthetic chemistry, for example, filtration, extraction, washing, drying, concentration, recrystallization, various kinds of chromatography, or the like. The intermediates can also be subjected to the subsequent reactions without purification.

For some of Compounds (I) and (IA), there may exist stereoisomers such as regioisomers, geometrical isomers, optical isomers and tautomers, and all possible isomers including them and mixtures thereof are included in the present invention.

When it is desired to obtain a salt of Compound (I) or (IA), in the case where Compound (I) or (IA) is obtained in the form of a salt, it can be purified as such, but in the case where it is obtained in a free state, it is dissolved or suspended in an appropriate solvent, and then adding an acid or a base thereto, and the resulting salt can be isolated and purified.

Further, Compounds (I) and (IA) and pharmaceutically acceptable salts thereof may exist in the form of adducts with water or various solvents, and these adducts can also be included in the present invention.

Specific examples of Compounds (I) and (IA) obtained according to the present invention are shown in Table 1. However, the compounds of the present invention should not be limited to these.

Table 1-1

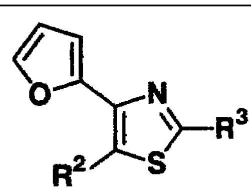 (I)

| Compound No. | $R^2$ | $R^3$ |
|---|---|---|
| 1 | 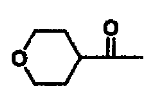 | 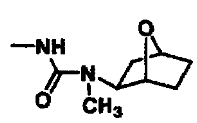 |

(continued)

| Compound No. | $R^2$ | $R^3$ |
|---|---|---|
| 2 | 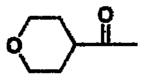 | 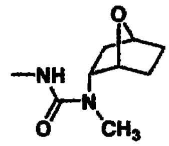 |
| 3 | 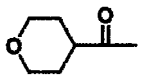 | 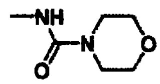 |
| 4 | 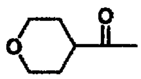 | 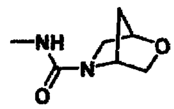 |
| 5 | 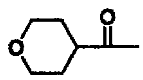 | 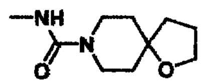 |
| 6 | 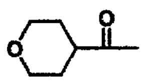 | 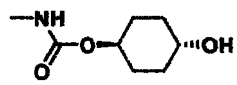 |
| 7 | 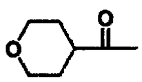 | 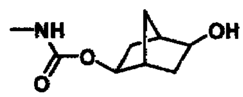 |
| 8 | 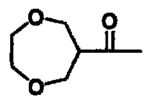 | 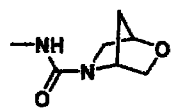 |
| 9 | 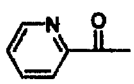 | 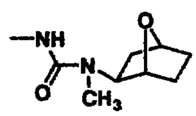 |
| 10 | 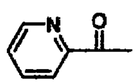 | 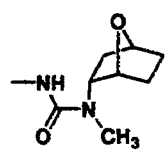 |

Table 1-2

| | | |
|---|---|---|
| 11 | 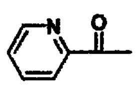 | 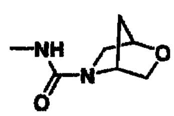 |
| 12 | 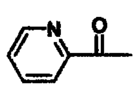 | 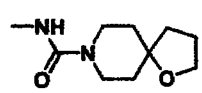 |
| 13 | 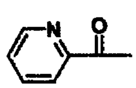 | 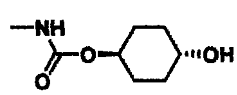 |

(continued)

| | | |
|---|---|---|
| 14 | 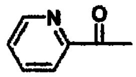 | 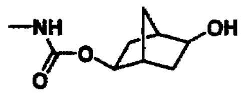 |
| 15 | 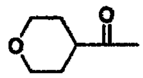 | 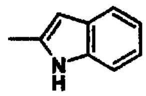 |
| 16 | 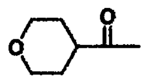 | 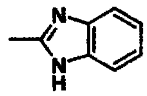 |
| 17 | 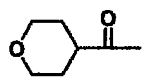 | 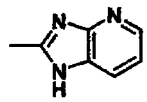 |
| 18 | 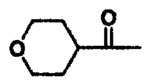 | 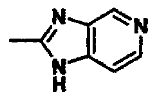 |
| 19 | 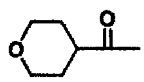 | 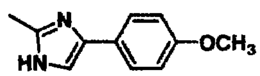 |
| 20 | 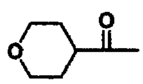 | 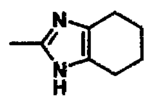 |
| 21 | 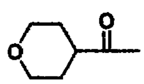 | 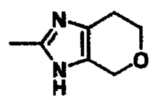 |
| 22 | 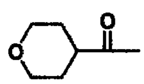 | 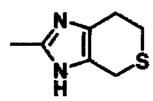 |
| 23 | 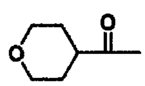 | 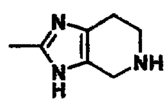 |

Table 1-3

| | | |
|---|---|---|
| 24 | 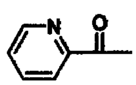 | 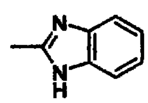 |
| 25 | 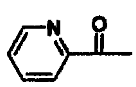 | 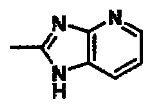 |
| 26 | 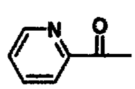 | 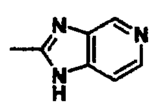 |

(continued)

| | | |
|---|---|---|
| 27 | 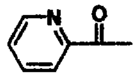 | 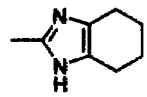 |
| 28 | 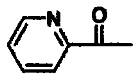 | 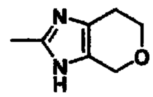 |
| 29 | 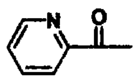 | 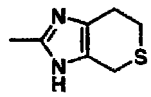 |
| 30 | 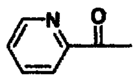 | 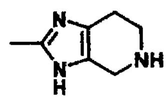 |
| 31 | 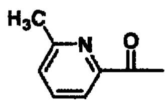 | 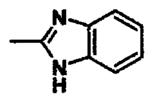 |
| 32 | 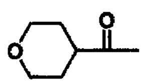 | 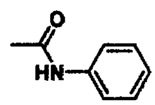 |
| 33 | 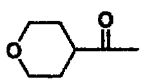 | 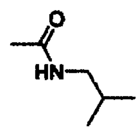 |
| 34 | 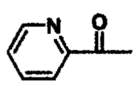 | 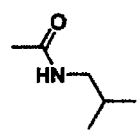 |

Pharmacological activities of typical Compounds (I) are illustrated below referring to Test Examples.

Test Example 1: Binding activity to adenosine receptor (adenosine $A_{2A}$ receptor binding test)

This test was carried out in a similar manner to a Bruns et al's method (Molecular Pharmacology, Vol. 29, p. 331, 1986). Corpus striatum of a rat (SD rat, by Nippon SLC) was suspended in 50 mL of an ice-cooled 50 mmol/L tris(hydroxymethyl) aminomethane hydrochloride (Tris-HCl) buffer (pH 7.7) using a polytron homogenizer (by Kinematica). The resulting suspension was centrifuged (48,000 x g, 20 minutes), and the resulting precipitate was resuspended by adding the same amount of a 50 mmol/L Tris-HCl buffer, followed by centrifugation under the same condition. The resulting final precipitate was suspended by adding a 50 mmol/L Tris-HCl buffer [containing 10 mmol/L magnesium chloride, adenosine deaminase 0.02 units/mg tissue (by Sigma)] thereto so that the tissue concentration was 5 mg (wet weight)/mL.

To 100 μL of the above purified cell suspension, 80 μL (final concentration: 6.0 mmol/L) of tritium-labeled CGS-21680 {$^3$H-2-[P-(2-carboxyethyl)phenethylamino]-5'-(N-ethylcarboxamido)-adenosine: 40 curies/mmol; by New England Nuclear [The Journal of Pharmacology and Experimental Therapeutics, Vol. 251, p. 888, 1989]} and 20μ L of a test compound solution ($10^{-7}$ mol/L; a solution of a test compound in DMSO was diluted with a Tris-HCl buffer) were added. The resulting mixture was allowed to stand at 25°C for 120 minutes, followed by rapid suction filtration through a glass fiber paper filter (GF/C; by Whatman). The filter was immediately washed three times with 200 μL of an ice-cooled 50 mmol/L Tris-HCl buffer. The glass fiber paper filter was transferred into a vial, Microscinti (by Perkin Elmer) was added thereto, and the radioactivity level was measured with Topcount (by Perkin Elmer).

The inhibition rate of the test compound to the adenosine $A_{2A}$ receptor binding ($^3$H-CGS21680 binding) was calculated according to the following equation.

$$\text{Inhibition rate (\%)} = \{1 - [(\text{Amount of binding in the presence of compound}) - (\text{Amount of non-specific binding})] / [(\text{Amount of total binding}) - (\text{Amount of non-specific binding})]\} \times 100$$

Amount of total binding means the $^{3}$H-CGS21680 binding radioactivity content in the absence of the test compound. Amount of non-specific binding means the $^{3}$H-CGS21680 binding radioactivity content in the presence of 100 μmol/L cyclopentyladenosine (CPA; by Sigma). Amount of binding in the presence of compound means the $^{3}$H-CGS21680 binding radioactivity content in the presence of $10^{-7}$ mol/L of the test compound.
The results are shown in Table 2.
Table 2

| Compound No. | Rat adenosine $A_{2A}$ receptor binding inhibition rate (%) ($10^{-7}$ mol/L) |
|---|---|
| 18 | 81 |
| 19 | 63 |
| 24 | 94 |
| 29 | 90 |
| 32 | 26 |
| 33 | 50 |

Table 2 indicates that Compounds (I) have a strong adenosine $A_{2A}$ receptor antagonism. Therefore, it was suggested that the pharmaceutical composition comprising Compound (I), as an active ingredient, is effective in various diseases associated with adenosine $A_{2A}$ receptor [for example, a central nervous system disease such as Parkinson's disease, Alzheimer's disease, progressive supranuclear palsy, AIDS encephalopathy, transmissible spongiform encephalopathy, multiple sclerosis, amyotrophic lateral sclerosis, Huntington's disease, multiple system atrophy, cerebral ischemia, attention deficit hyperactivity disorder, sleep disorder, intermittent claudication, diabetes, an anxiety disorder (such as panic attack and panic disorder, phobia, obsessive-compulsive disorder, posttraumatic stress disorder, acute stress disorder, generalized anxiety disorder, or anxiety caused by physical disorder or a substance), a mood disorder (such as depression, dysthymic disorder, or mood-circulatory disorder), restless legs syndrome (RLS), drug dependence (such as alcohol dependence), eating disorder, epilepsy, migraine, or chronic musculoskeletal system pain; an ischemic heart disease such as myocardial infarction or cerebral infarction, or the like].

Test Example 2: Effect on CGS21680-induced catalepsy

Parkinson's disease is motor dysfunction based on the degeneration and cell death of the nigrostriatal dopaminergic neuron. When CGS21680 (an adenosine $A_{2A}$ receptor agonist) is intracerebroventricularly administered, GABAergic inhibitory synaptic transmission in medium sized spiny neurons of the corpus striatum is directly suppressed via the adenosine $A_{2A}$ receptor (Journal of Neuroscience, Vol. 16, p. 605, 1996). Therefore, it is considered that the adenosine $A_{2A}$ receptor functions to promote the output of GABAergic neurons from the corpus striatum into the external segment of globus pallidus, and as a result, catalepsy is induced by the administration of CGS21680.
The experiment was carried out using 10 male ddY mice of 5 weeks of age (body weight, 22 to 25 g, by Nippon SLC) per group. CGS21680 (by Kyowa Hakko Kogyo) was dissolved in physiological saline (by Otsuka Pharmaceutical), and intracerebroventricularly injected to the mice in an amount of 10 μg/20 μL. The test compound was used by being suspended in distilled water containing 0.5 w/v% methyl cellulose 400 cP (hereinafter abbreviated as MC) (by Otsuka Pharmaceutical). At 30 minutes before the intracerebroventricular injection of CGS21680, a suspension containing the test compound or a solution not containing the test compound (0.5% MC-containing distilled water: control) was orally administered to the mice (0.1 mL per 10 g of mouse body weight), respectively. At 1 hour after the administration of the test compound, the degree of catalepsy was measured with respect to each of the mice such that a pair of only forelimbs and a pair of only hindlimbs of each mouse were placed by turns on a vertically installed acrylic stand with a height of 4.5 cm and a width of 1.0 cm. Each of the test compounds was orally administered at a dose of 10 mg/kg.
The judgment criteria of catalepsy scores are shown in the following Table 3.
Table 3: Judgment criteria of catalepsy scores

| Score | Duration of catalepsy |
|---|---|
| 0 | The duration of the posture in which the forelimbs were being placed on the stand was less than 5 seconds, and the duration of the posture in which the hindlimbs were being placed on the stand was less than 5 seconds. |
| 1 | The duration of the posture in which the forelimbs were being placed on the stand was less than 5 seconds, and the duration of the posture in which the hindlimbs were being placed on the stand was 5 seconds or more but less than 10 seconds; or the duration of the posture in which the forelimbs were being placed on the stand was 5 seconds or more but less than 10 seconds, and the duration of the posture in which the hindlimbs were being placed on the stand was less than 5 seconds. |
| 2 | The duration of the posture in which the forelimbs were being placed on the stand was 10 seconds or more, and the duration of the posture in which the hindlimbs were being placed on the stand was less than 5 seconds. |
| 3 | The duration of the posture in which the forelimbs were being placed on the stand was 5 seconds or more but less than 10 seconds, and the duration of the posture in which the hindlimbs were being placed on the stand was 5 seconds or more but less than 10 seconds; or the duration of the posture in which the forelimbs were being placed on the stand was less than 5 seconds, and the duration of the posture in which the hindlimbs were being placed on the stand was 10 seconds or more. |
| 4 | The duration of the posture in which the forelimbs were being placed on the stand was 5 seconds or more but less than 10 seconds, and the duration of the posture in which the hindlimbs were being placed on the stand was 10 seconds or more; or the duration of the posture in which the forelimbs were being placed on the stand was 10 seconds or more, and the duration of the posture in which the hindlimbs were being placed on the stand was 5 seconds or more but less than 10 seconds. |
| 5 | The duration of the posture in which the forelimbs were being placed on the stand was 10 seconds or more, and the duration of the posture in which the hindlimbs were being placed on the stand was 10 seconds or more. |

The catalepsy remission rate was calculated according to the following equation. When the remission rate was 80% or more, the compound was judged to have a catalepsy remission activity.

Remission rate (%) = [(Total score in the control group) – (Total score in the test compound

As a result, Compound 17 showed a catalepsy remission rate of 100% in the above test and was judged to have a catalepsy remission activity.

That is, it was suggested that Compound (I) is effective in treating and/or preventing Parkinson's disease.

Although Compounds (I) or pharmaceutically acceptable salts thereof can be administered singly as such, it is generally preferred to offer them in the form of various pharmaceutical preparations. Such pharmaceutical preparations are to be used in animals or humans.

The pharmaceutical preparations according to the present invention can comprise Compounds (I) or pharmaceutically acceptable salts thereof, as the active ingredient, alone or in combination with any other active ingredients for the therapy. These pharmaceutical preparations may be produced by any methods well known in the technical field of pharmaceutics by mixing the active ingredient with one or more pharmaceutically acceptable carriers.

It is desirable to select a route of administration that is most effective for the therapy, and examples thereof include oral administration and parenteral administration such as intravenous administration.

Examples of the dosage form include tablets, injections, and the like.

Preparations suitable for oral administration such as tablets can be produced using, for example, an excipient such as lactose or mannitol, a disintegrator such as starch, a lubricant such as magnesium stearate, a binder such as hydroxypropyl cellulose, a surfactant such as a fatty acid ester, a plasticizer such as glycerin, or the like.

Preparations suitable for parenteral administration preferably comprise a sterilized aqueous preparation containing an active compound which is isotonic to the recipient's blood. In the case of an injection, for example, a solution for injection is prepared using a carrier comprising a brine solution, a glucose solution, or a mixture of a brine solution and a glucose solution.

The parenteral preparations may also comprise one or more auxiliary components selected from the excipient, the disintegrator, the lubricant, the binder, the surfactant and the plasticizer described in the examples of the oral preparations and a diluent, a preservative, a flavor, and the like.

In the case where Compounds (I) and (IA) or pharmaceutically acceptable salts thereof are used for the above-mentioned purpose, in general, they are administered systemically or locally, and orally or parenterally. The dose and the administration frequency may vary depending on the administration form, the age and the body weight of the patient, and the nature and the severity of the symptom to be treated. In the case of oral administration, in general, it may be administered once to several times a day in a dose of 0.01 to 1000 mg/adult, preferably 0.05 to 500 mg/adult. In the case of parenteral administration such as intravenous administration, in general, it may be administered once to several times a day or continuously administered in a mode of intravenous administration for 1 to 24 hours a day, in a dose of 0.001 to 1000 mg/adult, preferably 0.01 to 300 mg/adult. However, the dose and the administration frequency may vary depending on various conditions mentioned above.

The present invention is described in detail below with reference to the following Examples, Reference Examples and Preparation Examples.

The proton nuclear magnetic resonance spectrum ($^1H$ NMR) used in Examples is determined at 270 MHz or 300 MHz. Some compounds could not clearly show an exchangeable proton in some conditions. The signal multiplicity expression is an ordinary one, for which "br" indicates an apparently broad signal.

Example 1

4-(2-Furyl)-2-(2-indolyl)thiazol-5-yl tetrahydropyran-4-yl ketone (Compound 15)

2-Bromo-4-(2-furyl)thiazol-5-yl tetrahydropyran-4-yl ketone (100 mg, 0.292 mmol) obtained in Reference Example 4 was dissolved in 1,4-dioxane (3 mL), and 1H-indole-2-boronic acid (101 mg, 0.627 mmol), tetrakis (triphenylphosphine)palladium(0) (28.4 mg, 0.0245 mmol) and sodium carbonate (104 mg, 0.982 mmol) were added thereto, and the mixture was stirred at 60°C for 1 hour. Water was added to the reaction mixture, and the mixture was extracted with ethyl acetate. The organic layer was dried over anhydrous magnesium sulfate, and then the solvent was removed in vacuo. The resulting residue was purified by silica gel column chromatography (hexane : ethyl acetate = 1 : 1), whereby the title Compound (17.0 mg, 15%) was obtained.

$^1H$ NMR ($CDCl_3$, δ ppm): 1.78-1.94 (m, 4H), 3.12-3.22 (m, 1H), 3.40-3.49 (m, 2H), 4.01-4.07 (m, 2H), 6.62 (dd, J = 1.8, 3.6 Hz, 1H), 7.13-7.16 (m, 2H), 7.28-7.34 (m, 1H), 7.40-7.44 (m, 1H), 7.50 (dd, J = 0.8, 3.6 Hz, 1H), 7.62 (dd, J = 0.8, 1.8 Hz, 1H), 7.65-7.68 (m, 1H), 9.32 (br s, 1H)

ESIMS m/z: $[M+H]^+$ 379

Example 2

2-(Benzimidazol-2-yl)-4-(2-furyl)thiazol-5-yl tetrahydropyran-4-yl ketone (Compound 16)

Step 1

Methyl tetrahydropyran-4-carboxylate (1.33 mL, 10.0 mmol) was dissolved in THF (20 mL), N,O-dimethylhydroxylamine hydrochloride (1.51 g, 15.5 mmol) was added thereto, and the mixture was stirred. A THF solution (15.0 mL, 30.0 mmol) of 2.0 mol/L isopropyl magnesium chloride was added dropwise to the reaction mixture at -30°C under an argon atmosphere, and the mixture was stirred at -5°C for 1 hour. Water was added to the reaction mixture, and the mixture was extracted with ethyl acetate. The organic layer was washed with brine and dried over anhydrous magnesium sulfate, and then the solvent was removed in vacuo. The resulting residue was purified by distillation under reduced pressure, whereby N-methoxy-N-methyl tetrahydropyran-4-carboxylic acid amide (1.00 g, 58%) was obtained.

Boiling point: 125 to 129°C/8.0 hPa

$^1H$ NMR ($CDCl_3$, δ ppm): 1.57-1.66 (m, 2H), 1.77-1.93 (m, 2H), 2.85-2.94 (m, 1H), 3.18 (s, 3H), 3.44 (ddd, J = 2.4, 11.9, 11.9 Hz, 2H), 3.69 (s, 3H), 4.00 (ddd, J = 2.4, 11.9, 11.9 Hz, 2H)

Step 2

2-(Benzimidazol-2-yl)-4-(2-furyl)thiazole (167 mg, 0.625 mmol) obtained in Reference Example 8 was dissolved in THF (5 mL), and an n-hexane solution (1.01 mL, 1.56 mmol) of 1.58 mol/L n-butyl lithium was added thereto at - 78˚C under an argon atmosphere, and the mixture was stirred at -78˚C for 10 minutes. A THF solution (1 mL) of N-methoxy-N-methyl tetrahydropyran-4-carboxylic acid amide (270 mg, 1.56 mmol) obtained in Step 1 was added dropwise thereto, and the mixture was stirred at room temperature for 1 hour. The reaction mixture was poured into a saturated aqueous solution of ammonium chloride, and the mixture was extracted with ethyl acetate. The organic layer was washed with brine and dried over anhydrous magnesium sulfate, and then the solvent was removed in vacuo. The resulting residue was purified by silica gel column chromatography (hexane : ethyl acetate = 1 : 1), whereby the title Compound (191 mg, 81%) was obtained. $^1$H NMR (DMSO-$d_6$, δ ppm): 1.51-1.67 (m, 4H), 3.21-3.38 (m, 3H), 3.80-3.86 (m, 2H), 6.74 (dd, J = 1.8, 3.5 Hz, 1H), 7.29-7.32 (m, 2H), 7.41 (d, J = 3.5 Hz, 1H), 7.63-7.66 (m, 2H), 7.94 (d, J = 1.8 Hz, 1H).
APCIMS m/z: $[M+H]^+$ 380
Melting point: 250 to 251˚C

Example 3

4-(2-Furyl)-2-(imidazo[4,5-b]pyridin-2-yl)thiazol-5-yl tetrahydropyran-4-yl ketone (Compound 17)

In the same manner as in Example 2, by using 4-(2-furyl)-2-(imidazo[4,5-b]pyridin-2-yl)thiazole (125 mg, 0.466 mmol) obtained in Reference Example 9 in place of 2-(benzimidazol-2-yl)-4-(2-furyl)thiazole, the title Compound (71.0 mg, 40%) was obtained.
$^1$H NMR (DMSO-$d_6$, δ ppm): 1.57-1.91 (m, 4H), 3.26-3.41 (m, 3H), 3.86-3.93 (m, 2H), 6.77 (dd, J = 1.8, 3.5 Hz, 1H), 7.33-7.39 (m, 1H), 7.41 (dd, J = 0.8, 3.5 H, 1H), 7.98 (dd, J = 0.8, 1.8 Hz, 1H), 8.07-8.10 (m, 1H), 8.47-8.50 (m, 1H).
APCIMS m/z: $[M+H]^+$ 381
Melting point: 265 to 268˚C (decomposition)

Example 4

4-(2-Furyl)-2-(imidazo[4,5-c]pyridin-2-yl)thiazol-5-yl tetrahydropyran-4-yl ketone (Compound 18)

In the same manner as in Example 2, by using 4-(2-furyl)-2-(imidazo[4,5-c]pyridin-2-yl)thiazole (137 mg, 0.511 mmol) obtained in Reference Example 10 in place of 2-(benzimidazol-2-yl)-4-(2-furyl)thiazole, the title Compound (7.00 mg, 4%) was obtained.
$^1$H NMR ($CDCl_3$, δ ppm): 1.89-1.95 (m, 4H), 3.18-3.21 (m, 1H), 3.42-3.52 (m, 2H), 4.04-4.11 (m, 2H), 6.61 (dd, J = 1.8, 3.5 Hz, 1H), 7.59-7.62 (m, 3H), 8.54 (d, J = 5.6 Hz, 1H), 9.19 (s, 1H).
APCIMS m/z: $[M+H]^+$ 381

Example 5

4-(2-Furyl)-2-[4-(4-methoxyphenyl)imidazol-2-yl]thiazol-5-yl tetrahydropyran-4-yl ketone (Compound 19)

In the same manner as in Example 2, by using 4-(2-furyl)-2-[4-(4-methoxyphenyl)imidazol-2-yl]thiazole (100 mg, 0.309 mmol) obtained in Reference Example 12 in place of 2-(benzimidazol-2-yl)-4-(2-furyl)thiazole, the title Compound (113 mg, 84%) was obtained.
$^1$H NMR (DMSO-$d_6$, δ ppm): 1.53-1.78 (m, 4H), 3.23-3.39 (m, 3H), 3.78 (s, 3H), 3.86-3.90 (m, 2H), 6.73 (dd, J = 1.7, 3.3 Hz, 1H), 6.96-6.99 (m, 2H), 7.42 (d, J = 3.3 Hz, 1H), 7.79-7.82 (m, 3H), 7.94 (d, J = 1.7 Hz, 1H).
APCIMS m/z: $[M+H]^+$ 436
Melting point: 178 to 180˚C

Example 6

4-(2-Furyl)-2-(4,5,6,7-tetrahydro-1H-benzimidazol-2-yl)-5-(tetrahydropyran-4-ylcarbonyl)thiazole (Compound 20)

In the same manner as in Example 2, by using 2-[4-(2-furyl-thiazole-2-yl)-4,5,6,7-tetrahydro-1H-benzimidazole (167 mg, 0.615 mmol) obtained in Reference Example 14 in place of 2-(benzimidazol-2-yl)-4-(2-furyl) thiazole, the title Compound (23.0 mg, 10%) was obtained.
$^1$H NMR ($CDCl_3$, δ ppm): 1.84-1.96 (m, 12H), 3.08-3.15 (m, 1H), 3.40-3.49 (m, 2H), 4.01-4.07 (m, 2H), 6.58 (dd, J =

1.7, 3.5 Hz, 1H), 7.58 (dd, J = 0.7, 1.7 Hz, 1H), 7.70 (dd, J = 0.7, 3.5 Hz, 1H)

Example 7

4-(2- Furyl)- 5-(tetrahydropyran- 4- ylcarbonyl)- 2-(3,4,6,7- tetrahydrothiopyrano [3,4- d] imidazol- 2- yl) thiazole (Compound 22)

In the same manner as in Example 2, by using 2-[4-(2-furyl-thiazole-2-yl)-3,4,6,7-tetrahydrothiopyrano[3,4-d]imidazole (133 mg, 0.460 mmol) obtained in Reference Example 15 in place of 2-(benzimidazol-2-yl)-4-(2-furyl)thiazole, the title Compound (17.0 mg, 9%) was obtained.

$^1$H NMR ($CDCl_3$, δ ppm): 1.72-1.92 (m, 4H), 2.97 (s, 4H), 3.09-3.15 (m, 1H), 3.41-3.49 (m, 2H), 3.76 (s, 2H), 4.03-4.07 (m, 2H), 6.60 (dd, J = 1.6, 3.3 Hz, 1H), 7.59 (d, J = 1.6 Hz, 1H), 7.72 (d, J = 3.3 Hz, 1H)

Example 8

4-(2-Furyl)-5-(pyridin-2-ylcarbonyl)-2-(2-benzimidazolyl)thiazole (Compound 24)

In the same manner as in Example 2, by using N-methoxy-N-methyl-pyridin-2-carboxylic acid amide in place of N-methoxy-N-methyl-tetrahydropyran-4-carboxylic acid amide, the title Compound (57.0 mg, 27%) was obtained from 2-(benzimidazol-2-yl)-4-(2-furyl) thiazole (150 mg, 0.561 mmol).

$^1$H NMR ($CDCl_3$, δ ppm): 6.71 (dd, J = 1.8, 3.5 Hz, 1H), 7.30-7.36 (m, 2H), 7.48 (dd, J = 0.7, 3.5 Hz, 1H), 7.59-7.61 (m, 1H), 7.74-7.78 (m, 2H), 7.84 (dd, J = 0.7, 1.8 Hz, 1H), 8.13-8.20 (m, 2H), 8.78-8.80 (m, 1H), 13.6 (br s, 1H)

Example 9

4-(2-Furyl)-5-(pyridin-2-ylcarbonyl)-2-(3,4,6,7-tetrahydrothiopyrano[3,4-d]imidazol-2-yl)thiazole (Compound 29)

In the same manner as in Example 2, by using N-methoxy-N-methyl-pyridin-2-carboxylic acid amide in place of N-methoxy-N-methyl-tetrahydropyran-4-carboxylic acid amide, and by using 2-[4-(2-furyl-thiazole-2-yl)-3,4,6,7-tetrahydrothiopyrano[3,4-d]imidazole (137 mg, 0.473 mmol) obtained in Reference Example 15 in place of 2-(benzimidazol-2-yl)-4-(2-furyl)thiazole, the title Compound (36.0 mg, 19%) was obtained.

$^1$H NMR ($CDCl_3$, δ ppm): 2.95 (m, 4H), 3.76-3.78 (m, 2H), 6.59 (dd, J = 1.7, 3.5 Hz, 1H), 7.51-7.54 (m, 2H), 7.78 (dd, J = 0.7, 3.5 Hz, 1H), 7.88-7.95 (m, 1H), 8.21-8.24 (m, 1H), 8.69-8.71 (m, 1H)

Example 10

4-(2-Furyl)-5-(tetrahydropyran-4-ylcarbonyl)thiazol-2-carboxylic acid phenylamide (Compound 32)

In the same manner as in Example 2, by using 4-(2-furyl)thiazol-2-carboxylic acid phenylamide (120 mg, 0.444 mmol) obtained in Reference Example 16 in place of 2-(benzimidazol-2-yl)-4-(2-furyl)thiazole, the title Compound (18.0 mg, 11%) was obtained.

$^1$H NMR ($CDCl_3$, δ ppm): 1.80-1.92 (m, 4H), 3.12-3.23 (m, 1H), 3.40-3.50 (m, 2H), 4.01-4.07 (m, 2H), 6.62 (dd, J = 1.8, 3.6 Hz, 1H), 7.21-7.24 (m, 1H), 7.38-7.48 (m, 3H), 7.62 (d, J = 1.8 Hz, 1H), 7.72-7.75 (m, 2H), 9.12 (br s, 1H)

Example 11

4-(2-Furyl)-5-(tetrahydropyran-4-ylcarbonyl)thiazol-2-carboxylic acid isobutylamide (Compound 33)

In the same manner as in Example 2, by using 4-(2-furyl)thiazol-2-carboxylic acid isobutylamide (186 mg, 0.743 mmol) obtained in Reference Example 17 in place of 2-(benzimidazol-2-yl)-4-(2-furyl)thiazole, the title Compound (32.0 mg, 12%) was obtained.

$^1$H NMR ($CDCl_3$, δ ppm): 1.00 (d, J = 6.8 Hz, 6H), 1.82-2.05 (m, 5H), 3.12-3.16 (m, 1H), 3.29-3.49 (m, 4H), 3.99-4.06 (m, 2H), 6.59 (dd, J = 1.7, 3.5 Hz, 1H), 7.44 (dd, J = 0.8, 3.5 Hz, 1H), 7.59 (dd, J = 0.8, 1.7 Hz, 1H)

Example 12

4-(2-Furyl)-5-(pyridin-2-ylcarbonyl)thiazol-2-carboxylic acid isobutylamide (Compound 34)

In the same manner as in Example 2, by using N-methoxy-N-methyl-pyridin-2-carboxylic acid amide in place of N-methoxy-N-methyl-tetrahydropyran-4-carboxylic acid amide, and by using 4-(2-furyl)thiazol-2-carboxylic acid isobutylamide (241 mg, 0.963 mmol) obtained in Reference Example 17 in place of 2-(benzimidazol-2-yl)-4-(2-furyl)thiazole, the title Compound (172 mg, 50%) was obtained.

$^1$H NMR ($CDCl_3$, δ ppm): 1.00 (d, J = 6.8 Hz, 6H), 1.92-1.97 (m, 1H), 3.29-3.34 (m, 2H), 6.54 (dd, J = 1.7, 3.5 Hz, 1H), 7.45-7.54 (m, 3H), 7.88-7.94 (m, 1H), 8.19-8.22 (m, 1H), 8.65-8.67 (m, 1H)

[Reference Example 1]

2-Amino-5-bromo-4-(2-furyl)thiazole (Compound a)

Step 1

2-Acetylfuran (5.10 g, 46.0 mmol) was dissolved in a mixed solvent of dichloromethane (50 mL) and methanol (50 mL), and N,N,N,N-tetra-n-butyl ammonium tribromide (22.3 g, 46.0 mmol) was added thereto, and the mixture was stirred at room temperature for 1 hour. The reaction mixture was concentrated under reduced pressure, water was added to the resulting residue, and the mixture was extracted with ethyl acetate. The organic layer was washed with brine and dried over anhydrous magnesium sulfate, and then the solvent was removed in vacuo. The resulting residue was dissolved in acetonitrile (60 mL), thiourea (3.5 g, 46.0 mmol) was added thereto, and the mixture was stirred at room temperature

for 30 minutes. The precipitated solid was collected by filtration, and the resulting solid was dissolved in a mixed solvent of a saturated aqueous solution of sodium hydrogen carbonate and ethyl acetate, and extracted. The organic layer was washed with brine and dried over anhydrous magnesium sulfate, and then the solvent was removed in vacuo. The resulting residue was purified by silica gel column chromatography (hexane : ethyl acetate = 3 : 1), whereby 2-amino-4-(2-furyl)thiazole (1.53 g, 20%) was obtained.

$^1$H NMR ($CDCl_3$, $\delta$ ppm): 5.17 (br s, 2H), 6.43 (dd, J = 2.0, 3.3 Hz, 1H), 6.61 (d, J = 3.3 Hz, 1H), 6.69 (s, 1H), 7.49 (d, J = 2.0 Hz, 1H)

Step 2

2-Amino-4-(2-furyl)thiazole (330 mg, 1.99 mmol) obtained in Step 1 was suspended in chloroform (4 mL), and N-bromosuccinimide (360 mg, 2.02 mmol) was added thereto, and the mixture was stirred at room temperature for 1 hour. Water was added to the reaction mixture, and the mixture was extracted with ethyl acetate. The organic layer was washed with brine and dried over anhydrous magnesium sulfate, and then the solvent was removed in vacuo, whereby the title Compound (438 mg, 90%) was obtained.

$^1$H NMR ($CDCl_3$, $\delta$ ppm): 5.08 (br s, 2H), 6.48 (dd, J = 2.0, 3.3 Hz, 1H), 6.96 (d, J = 3.3 Hz, 1H), 7.48 (d, J = 2.0 Hz, 1H)

[Reference Example 2]

tert-Butyl N-[5-bromo-4-(2-furyl)thiazol-2-yl] carbamate (Compound b)

Compound a (12.0 g, 49.0 mmol) obtained in Reference Example 1, di-tert-butyl dicarbonate (21.3 g, 97.9 mmol), triethylamine (17.1 mL, 122 mmol) and N,N-dimethylaminopyridine (0.60 g, 4.91 mmol) were dissolved in DMI (200 mL), and the mixture was stirred overnight at room temperature. Water was added to the reaction mixture, and the mixture was extracted with ethyl acetate. The organic layer was washed with brine and dried over anhydrous magnesium sulfate, and then the solvent was removed in vacuo. The resulting residue was purified by silica gel column chromatography (hexane : ethyl acetate = 5 : 1), whereby the title Compound (14.2 g, 84%) was obtained.

$^1$H NMR (DMSO-$d_6$, $\delta$ ppm): 1.49 (s, 9H), 6.64 (dd, J = 2.0, 3.3 Hz, 1H), 6.91 (dd, J = 0.7, 3.3 Hz, 1H), 7.80 (dd, J = 0.7, 2.0 Hz, 1H)

[Reference Example 3]

2-Amino-4-(2-furyl)-5-(tetrahydropyran-4-ylcarbonyl)thiazole (Compound c)

Step 1

Tetrahydropyran-4-carboxylic acid (1.00 g, 7.69 mmol), phenol (651 mg, 6.92 mmol) and (benzothiazol-1-yloxy)tripyrrolidinophosphonium hexafluorophosphate (PyBOP; 4.40 g, 8.45 mmol) were dissolved in DMF (15 mL), triethylamine (2.36 mL, 16.9 mmol) was added thereto, and the mixture was stirred at room temperature for 4 hours. The reaction mixture was poured into water, and the mixture was extracted with ethyl acetate. The organic layer was sequentially washed with a saturated aqueous solution of sodium hydrogen carbonate and brine, and dried over anhydrous magnesium sulfate, and then the solvent was removed in vacuo. The resulting residue was purified by silica gel column chromatography (hexane : ethyl acetate = 3 : 2), whereby phenyl tetrahydropyran-4-carboxylate (1.28 g, 81%) was obtained.

$^1$H NMR ($CDCl_3$, $\delta$ ppm): 1.62-1.79 (m, 2H), 1.86-1.96 (m, 2H), 2.89 (tt, J = 4.6, 11.0 Hz, 1H), 3.42 (ddd, J = 2.4, 11.0, 11.0 Hz, 2H), 3.88 (ddd, J = 2.4, 11.0, 11.0 Hz, 2H), 7.09-7.16 (m, 2H), 7.22-7.31 (m, 1H), 7.37-7.44 (m, 2H)

Step 2

Compound b (1.04 g, 3.00 mmol) obtained in Reference Example 2 was dissolved in THF (8 mL), and an n-hexane solution (4.20 mL, 6.61 mmol) of 1.58 mol/L n-butyl lithium was added thereto at -78˚C under an argon atmosphere, and the mixture was stirred at -78˚C for 10 minutes. Phenyl tetrahydropyran-4-carboxylate (620 mg, 3.00 mmol) obtained in Step 1 was added dropwise to the reaction mixture, and the mixture was stirred at room temperature for 1 hour. The reaction mixture was poured into a saturated aqueous solution of ammonium chloride, and the mixture was extracted with ethyl acetate. The organic layer was washed with brine and dried over anhydrous magnesium sulfate, and then the solvent was removed in vacuo. The resulting residue was purified by silica gel column chromatography (hexane : ethyl acetate = 3 : 2), whereby tert-butyl N-[4-(2-furyl)-5-(tetrahydropyran-4-ylcarbonyl)thiazole-2-yl] carbamate (350 mg, 35%) was obtained.

$^1$H NMR ($CDCl_3$, $\delta$ ppm): 1.53 (s, 9H), 1.76-1.94 (m, 4H), 3.06-3.18 (m, 1H), 3.46 (ddd, J = 2.7, 11.6, 11.6 Hz, 2H), 4.03 (ddd, J = 2.7, 4.0. 11.6 Hz, 2H), 6.55 (dd, J = 1.9, 3.5 Hz, 1H), 7.55 (dd, J = 0.8, 1.9 Hz, 1H), 7.76 (dd, J = 0.8, 3.5 Hz, 1H), 8.68 (br s, 1H)

Step 3

tert-Butyl N-[4-(2-furyl)-5-(tetrahydropyran-4-ylcarbonyl)thiazole-2-yl]carbamate (350 mg, 1.05 mmol) obtained in Step 2 was dissolved in trifluoroacetic acid (5 mL), and the mixture was stirred at room temperature for 1 hour. The reaction mixture was concentrated under reduced pressure, and ethyl acetate and a saturated aqueous solution of sodium hydrogen carbonate were added to the resulting residue, and the organic layer was separated. The organic layer was washed with brine and dried over anhydrous magnesium sulfate, and then the solvent was removed in vacuo. The resulting residue was purified by silica gel column chromatography (hexane : ethyl acetate = 1 : 4), whereby the title Compound (212 mg, 72%) was obtained.

$^1H$ NMR (DMSO-$d_6$, δ ppm): 1.52-1.64 (m, 4H), 2.89-3.03 (m, 1H), 3.24 (ddd, J = 3.8, 11.3, 11.3 Hz, 2H), 3.85 (ddd, J = 2.7, 3.8, 11.3 Hz, 2H), 6.65 (dd, J = 1.9, 3.5 Hz, 1H), 7.24 (d, J = 3.5 Hz, 1H), 7.84 (d, J = 1.9 Hz, 1H), 7.96 (br s, 2H)

[Reference Example 4]

2-Bromo-4-(2-furyl)thiazol-5-yl tetrahydropyran-4-yl ketone

Compound c (163 mg, 0.600 mmol) obtained in Reference Example 3 was dissolved in acetonitrile (5 mL), and isoamyl nitrite (0.242 mL, 1.80 mmol) and copper(II) bromide (134 mg, 0.600 mmol) were added thereto, and the mixture was stirred at 60˚C for 1 hour. The reaction mixture was concentrated under reduced pressure, and the resulting residue was purified by silica gel column chromatography (hexane : ethyl acetate = 3 : 1), whereby the title Compound (85.0 mg, 41%) was obtained.

$^1H$ NMR ($CDCl_3$, δ ppm): 1.75-1.92 (m, 4H), 3.11-3.18 (m, 1H), 3.36-3.45 (m, 2H), 3.98-4.05 (m, 2H), 6.58 (dd, J = 1.7, 3.6 Hz, 1H), 7.39 (dd, J = 0.7, 3.6 Hz, 1H), 7.58 (dd, J = 0.7, 1.7 Hz, 1H)

[Reference Example 5]

(2-Furoyl)methylthiocyanate

2-Acetyl furan (10.0 mL, 100 mmol) was dissolved in ethanol (200 mL), and N,N,N,N-tetra-n-butyl ammonium tribromide (50.6 g, 105 mmol) was added thereto, and the mixture was stirred at 50˚C for 1 hour. Sodium thiocyanate (8.91 g, 110 mmol) was added to the reaction mixture, and the mixture was stirred for an additional 1 hour. A saturated aqueous solution of sodium hydrogen carbonate was added to the reaction mixture, and the mixture was extracted with ethyl acetate. The organic layer was dried over anhydrous magnesium sulfate, and then the solvent was removed in vacuo. The precipitated crystal was collected by filtration, whereby the title Compound (11.2 g, 67%) was obtained.

$^1H$ NMR ($CDCl_3$, δ ppm): 4.44 (s, 2H), 6.65 (dd, J = 1.6, 3.7 Hz, 1H), 7.38 (dd, J = 0.7, 3.7 Hz, 1H), 7.68 (dd, J = 0.7, 1.6 Hz, 1H)

[Reference Example 6]

2-Bromo-4-(2-furyl)thiazole

(2-Furoyl)methylthiocyanate (896 mg, 5.20 mmol) obtained in Reference Example 5 was dissolved in ethyl acetate (25 mL), and hydrogen bromide/acetic acid solution (1.85 mL, 10.4 mmol) was added thereto at 0˚C under an argon atmosphere, and the mixture was stirred at room temperature for 2 hours. The reaction mixture was concentrated under reduced pressure, and a saturated aqueous solution of sodium hydrogen carbonate was added thereto, and the mixture was extracted with ethyl acetate. The organic layer was dried over anhydrous magnesium sulfate, and then the solvent was removed in vacuo. The resulting residue was purified by silica gel column chromatography (hexane : ethyl acetate = 3 : 1), whereby the title Compound (814 mg, 68%) was obtained.

$^1H$ NMR ($CDCl_3$, δ ppm): 6.48 (dd, J = 1.7, 3.3 Hz, 1H), 6.83 (dd, J = 0.7, 3.3 Hz, 1H), 7.34 (s, 1H), 7.43 (dd, J = 0.7, 1.7 Hz, 1H)

[Reference Example 7]

2-Formyl-4-(2-furyl)thiazole

2-Bromo-4-(2-furyl)thiazole (3.00 g, 13.0 mmol) obtained in Reference Example 6 was dissolved in THF (65 mL), and an n-hexane solution (9.11 mL, 14.3 mmol) of 1.58 mol/L n-butyl lithium was added thereto at -78˚C under an argon atmosphere, and the mixture was stirred at -78˚C for 10 minutes. DMF (2.02 mL, 26.1 mmol) was added dropwise thereto, and the mixture was stirred at room temperature for 1 hour. The reaction mixture was poured into a saturated aqueous solution of ammonium chloride, and the mixture was extracted with ethyl acetate. The organic layer was washed with brine and dried over anhydrous magnesium sulfate, and then the solvent was removed in vacuo. The resulting residue was purified by silica gel column chromatography (hexane : ethyl acetate = 3 : 1), whereby the title Compound (1.49 g, 64%) was obtained.

$^1H$ NMR ($CDCl_3$, δ ppm): 6.54 (dd, J = 1.8, 3.5 Hz, 1H), 6.93 (dd, J = 0.7, 3.5 Hz, 1H), 7.51 (dd, J = 0.7, 1.8 Hz, 1H), 7.81 (s, 1H), 10.04 (s, 1H)

[Reference Example 8]

2-(Benzimidazol-2-yl)-4-(2-furyl)thiazole

2-Formyl-4-(2-furyl)thiazole (116 mg, 0.647 mmol) obtained in Reference Example 7 was dissolved in nitrobenzene (5 mL), and orthophenylenediamine (77.0 mg, 0.712 mmol) was added thereto, and the mixture was stirred at 150˚C for 10 hours. The reaction mixture was purified by silica gel column chromatography (chloroform : methanol = 95 : 5) as such, whereby the title Compound (81.0 mg, 47%) was obtained.

$^1H$ NMR ($CDCl_3$, δ ppm): 6.53 (dd, J = 1.8, 3.5 Hz, 1H), 6.86-6.88 (d, J = 3.5 Hz, 1H), 7.26-7.34 (m, 2H), 7.49-7.58 (m, 3H), 7.85 (d, J = 1.8 Hz, 1H)

[Reference Example 9]

4-(2-Furyl)-2-(imidazo[4,5-b]pyridin-2-yl)thiazole

In the same manner as in Reference Example 8, by using 2,3-diaminopyridine in place of orthophenylenediamine, the title Compound (148 mg, 49%) was obtained from 2-formyl-4-(2-furyl)thiazole (200 mg, 1.12 mmol).

$^1H$ NMR (DMSO-$d_6$, δ ppm): 6.69 (dd, J = 1.7, 3.3 Hz, 1H), 6.98 (d, J = 3.3 Hz, 1H), 7.32-7.39 (m, 1H), 7.84 (d, J = 1.7 Hz, 1H), 8.07-8.11 (m, 1H). 8.12 (s, 1H), 8.46-8.48 (m, 1H)

[Reference Example 10]

4-(2-Furyl)-2-(imidazo[4,5-c]pyridin-2-yl)thiazole

In the same manner as in Reference Example 8, by using 3,4-diaminopyridine in place of orthophenylenediamine, the title Compound (137 mg, 46%) was obtained from 2-formyl-4-(2-furyl)thiazole (200 mg, 1.12 mmol).

$^1$H NMR (DMSO-$d_6$, δ ppm): 6.68 (dd, J = 1.8, 3.3 Hz, 1H), 6.97 (d, J = 3.3 Hz, 1H), 7.60-7.63 (m, 1H), 7.84 (d, J = 1.8 Hz, 1H), 8.11 (s, 1H), 8.35-8.37 (m, 1H), 9.00-9.01 (m, 1H)

[Reference Example 11]

4-(2-Furyl)thiazol-2-thioamide

In the same manner as in Reference Example 5, by using rubeanic acid in place of sodium thiocyanate, the title Compound (1.01 g, 48%) was obtained from 2-acetyl furan (1.00 mL, 10.0 mmol).

$^1$H NMR (DMSO-$d_6$, δ ppm): 6.64 (dd, J = 1.7, 3.3 Hz, 1H), 6.91 (dd, J = 0.7, 3.3 Hz, 1H), 7.79 (dd, J = 0.7, 1.7 Hz, 1H), 8.06 (s, 1H), 10.21 (br s, 2H)

[Reference Example 12]

4-(2-Furyl)-2-[4-(4-methoxyphenyl)imidazol-2-yl]thiazole

4-(2-Furyl)thiazol-2-thioamide (210 mg, 1.00 mmol) obtained in Reference Example 11 was dissolved in THF (25 mL), and methyl iodide (5 mL) was added thereto, and the mixture was stirred at room temperature for 3 days. The reaction mixture was concentrated under reduced pressure, and 2-amino-4'-methoxyacetophenone hydrochloride (807 mg, 4.00 mmol) was added thereto, and the mixture was stirred overnight at 50˚C. A saturated aqueous solution of sodium hydrogen carbonate was added to the reaction mixture, and the mixture was extracted with ethyl acetate. The organic layer was dried over anhydrous magnesium sulfate, and then the solvent was removed in vacuo. The resulting residue was purified by silica gel column chromatography (chloroform : methanol = 97 : 3), whereby the title Compound (105 mg, 31%) was obtained.

$^1$H NMR (DMSO-$d_6$, δ ppm): 3.78 (s, 3H), 6.65 (dd, J = 1.8, 3.3 Hz, 1H), 6.89 (d, J = 3.3 Hz, 1H), 6.92-7.02 (m, 2H), 7.73-7.85 (m, 5H)

[Reference Example 13]

Ethyl 4-(2-furyl)thiazol-2-carboxylate

In the same manner as in Reference Example 5, by using ethylthiooxamate in place of sodium thiocyanate, the title Compound (2.55 g, 30%) was obtained from acetyl furan (3.77 mL, 37.5 mmol).

$^1$H NMR ($CDCl_3$, δ ppm): 1.46 (t, J = 7.1 Hz, 3H), 4.51 (q, J = 7.1 Hz, 1H), 6.51 (dd, J = 1.7, 3.3 Hz, 1H), 6.98 (d, J = 3.3 Hz, 1H), 7.47 (d, J = 1.7 Hz, 1H), 7.68 (s, 1H)

[Reference Example 14]

2-[4-(2-Furyl-thiazole-2-yl)-4,5,6,7-tetrahydro-1H-benzimidazole

Step 1

Ethyl 4-(2-furyl)thiazol-2-carboxylate (335 mg, 1.50 mmol) obtained in Reference Example 13 was dissolved in methanol, and an aqueous solution of 4 mol/L sodium hydroxide (2.5 mL) was added thereto, and the mixture was stirred at 50˚C for 1 hour. The reaction mixture was neutralized with hydrochloric acid, and then the solvent was removed in vacuo. The resulting residue was dissolved in DMF (5 mL), and 2-aminocyclohexanol (518 mg, 4.50 mmol), EDC hydrochloride (864 mg, 4.50 mmol), 1-hydroxybenzotriazole monohydrate (68.9 mg, 4.50 mmol) were added thereto, and the mixture was stirred at room temperature for 3 hours. Water and ethyl acetate were added to the reaction mixture to extract the mixture. The organic layer was washed with brine and dried over anhydrous magnesium sulfate, and then the solvent was removed in vacuo. The resulting residue was purified by silica gel column chromatography (hexane : ethyl acetate = 1 : 1), whereby 4-(2-furyl)thiazol-2-carboxylic acid (2-hydroxycyclohexyl)amide (413 mg, 94%) was obtained.

$^1$H NMR ($CDCl_3$, δ ppm): 1.26-1.49 (m, 4H), 1.74-1.81 (m, 2H), 2.04-2.16 (m, 2H), 3.48-3.55 (m, 1H), 3.78-4.00 (m, 1H), 6.51 (dd, J = 1.8, 3.3 Hz, 1H), 6.81 (dd, J = 0.7, 3.3 Hz, 1H), 7.48 (dd, J = 0.7, 1.8 Hz, 1H), 7.61 (s, 1H)

Step 2

Oxaryl chloride (0.776 mL, 1.55 mol) and dimethylsulfoxide (0.240 mL, 3.38 mmol) were dissolved in dichloromethane (3 mL), and the mixture was stirred at - 60˚C for 10 minutes. Then, 4-(2-furyl)thiazol-2-carboxylic acid (2-hydroxycyclohexyl)amide (413 mg, 1.41 mmol) obtained in Step 1 was added thereto, and the mixture was stirred for an additional 15 minutes. Triethylamine (0.983 mL, 7.05 mmol) was added thereto, and the mixture was stirred at room temperature for 10 minutes. Then, water and ethyl acetate were added thereto to extract the mixture. The organic layer was washed with brine and dried over anhydrous magnesium sulfate, and then the solvent was removed in vacuo. The resulting residue was purified by silica gel column chromatography (hexane : ethyl acetate = 1 : 1), whereby 4-(2-furyl)thiazol-2-carboxylic acid (2-oxocyclohexyl) amide (339 mg, 83%) was obtained.

$^1$H NMR ($CDCl_3$, δ ppm): 1.54-1.96 (m, 4H), 2.16-2.19 (m, 1H), 2.42-2.78 (m, 3H), 4.62-4.72 (m, 1H), 6.52 (dd, J = 1.8, 3.3 Hz, 1H), 6.90 (dd, J = 0.7, 3.3 Hz, 1H), 7.47 (dd, J = 0.7, 1.8 Hz, 1H), 7.62 (s, 1H), 8.12-8.14 (m, 1H)

Step 3

After 4-(2-furyl)thiazol-2-carboxylic acid (2-oxocyclohexyl)amide (239 mg, 0.823 mmol) obtained in Step 2 and ammonium trifluoroacetate (1.00 g, 7.63 mmol) were stirred at 140˚C for 2 hours, water and ethyl acetate were added thereto to

extract the mixture. The organic layer was washed with brine and dried over anhydrous magnesium sulfate, and then the solvent was removed in vacuo. The resulting residue was purified by silica gel column chromatography (hexane : ethyl acetate = 1 : 1), whereby the title Compound (63.0 mg, 28%) was obtained.

$^1$H NMR ($CDCl_3$, δ ppm): 1.81-1.86 (m, 4H), 2.57-2.65 (m, 4H), 6.48 (dd, J = 1.8, 3.3 Hz, 1H), 6.76 (dd, J = 0.7, 3.3 Hz, 1H), 7.31 (s, 1H), 7.46 (dd, J = 0.7, 1.8 Hz, 1H)

[Reference Example 15]

2-[4-(2-Furyl-thiazole-2-yl)-3,4,6,7-tetrahydrothiopyrano[3,4-d]imidazole

Step 1

4-Oxothiane (2.32 g, 20.0 mmol) was dissolved in ethanol, and hydroxylamine hydrochloride (2.78 g, 40.0 mmol) and potassium carbonate (5.52 g, 40.0 mmol) were added thereto, and the mixture was heated to reflux for 1 hour. Water and ethyl acetate were added to the reaction system to extract the mixture. The organic layer was washed with brine and dried over anhydrous magnesium sulfate, and then the solvent was removed in vacuo. The resulting residue was dissolved in pyridine (20 mL), and p-toluene sulfonic acid chloride (6.46 g, 24.0 mmol) was added thereto at -20˚C, and the mixture was stirred at - 20˚C for 1 hour. Water and ethyl acetate were added to the reaction system to extract the mixture. The organic layer was washed with brine and dried over anhydrous magnesium sulfate, and then the solvent was removed in vacuo. The resulting residue was dissolved in ethanol, and an ethanol solution of 4 mol/L potassium ethoxide (15.7 mL, 40.0 mmol) was added thereto at 0˚C, and the mixture was stirred for 1 hour. After the mixture was stirred at 50˚C for an additional 1 hour, water and ethyl acetate were added to the reaction system to extract the mixture. The organic layer was washed with brine and dried over anhydrous magnesium sulfate, and then the solvent was removed in vacuo. The resulting residue was purified by silica gel column chromatography (ethyl acetate), whereby 3-amino-4,4-diethoxythiopyrane (2.31 g, 56%) was obtained.

$^1$H NMR ($CDCl_3$, δ ppm): 1.19 (t, J = 7.1 Hz, 6H), 1.85-1.98 (m, 2H), 2.28-2.37 (m, 1H), 2.50-2.57 (m, 1H), 2.69-2.84 (m, 1H), 3.14-3.28 (m, 2H), 3.42 (q, J = 7.1 Hz, 4H)

Step 2

4-(2-Furyl)thiazol-2-thioamide (210 mg, 1.00 mmol) obtained in Reference Example 11 was dissolved in THF (25 mL), and methyl iodide (5 mL) was added thereto, and the mixture was stirred at room temperature for 3 days. After the solvent was removed in vacuo, 3-amino-4,4-diethoxythiopyrane (821 mg, 4.00 mmol) obtained in Step 1 was added thereto, and the mixture was stirred overnight at 50˚C. The solvent was removed in vacuo, and the resulting residue was dissolved in 6 mol/L hydrochloric acid (2 mL), and the mixture was stirred at room temperature for 1 hour. After the reaction mixture was neutralized with sodium hydroxide, water and ethyl acetate were added thereto to extract the mixture. After the organic layer was dried over anhydrous magnesium sulfate, the solvent was removed in vacuo. The residue obtained by distilling off the solvent under reduced pressure was purified by silica gel column chromatography (chloroform : methanol = 97 : 3), whereby the title Compound (270 mg, 93%) was obtained.

$^1$H NMR ($CDCl_3$, δ ppm): 2.88-3.25 (m, 4H), 3.72-3.90 (m, 2H), 6.48 (dd, J = 1.8, 3.3 Hz, 1H), 6.75 (d, J = 3.3 Hz, 1H), 7.41 (s, 1H), 7.46 (d, J = 1.8 Hz, 1H)

[Reference Example 16]

4-(2-Furyl)thiazol-2-carboxylic acid phenylamide

Ethyl 4-(2-furyl)thiazol-2-carboxylate (223 mg, 1.00 mmol) obtained in Reference Example 13 was dissolved in methanol, and an aqueous solution of 4 mol/L sodium hydroxide (2 mL) was added thereto, and the mixture was stirred at 50˚C for 1 hour. After the reaction mixture was neutralized with hydrochloric acid, the solvent was removed in vacuo. The resulting residue was dissolved in DMF (5 mL), and aniline (0.273 mL, 3.00 mmol), EDC hydrochloride (576 mg, 3.00 mmol), 1-hydroxybenzotriazole monohydrate (459 mg, 3.00 mmol) were added thereto, and the mixture was stirred at room temperature for 3 hours. Water was added to the reaction mixture, and the precipitated crystal was filtered, whereby the title Compound (242 mg, 90%) was obtained.

$^1$H NMR ($CDCl_3$, δ ppm): 6.54 (dd, J = 1.8, 3.5 Hz, 1H), 6.90 (dd, J = 0.7, 3.5 Hz, 1H), 7.16-7.22 (m, 1H), 7.38-7.43 (m, 2H), 7.50 (dd, J = 0.7, 1.8 Hz, 1H), 7.69 (s, 1H), 7.73-7.76 (m, 1H)

[Reference Example 17]

4-(2-Furyl)thiazol-2-carboxylic acid isobutylamide

In the same manner as in Reference Example 16, by using isobutylamine in place of aniline, the title Compound (537 mg, 96%) was obtained from ethyl 4-(2-furyl)thiazol-2-carboxylate (500 mg, 2.24 mmol).

$^1$H NMR ($CDCl_3$, δ ppm): 0.98-1.02 (m, 6H), 1.82-2.04 (m, 1H), 3.29-3.34 (m, 2H), 6.51 (dd, J = 1.8, 3.5 Hz, 1H), 6.82 (d, J = 3.5 Hz, 1H), 7.47 (d, J = 1.8 Hz, 1H), 7.62 (s, 1H)

Preparation Example 1: Tablet (Compound 18)

In a conventional method, tablets having the following composition are prepared. Compound 18 (40 g), lactose (286.8 g) and potato starch (60 g) are mixed, and a 10 % aqueous solution (120 g) of hydroxypropyl cellulose is added thereto. The mixture is kneaded in a conventional manner, granulated and dried, and dressed to give granules for tabletting. Magnesium stearate (1.2 g) is added thereto and mixed therein, and the mixture is tabletted using a tabletting machine with a pestle having a diameter of 8 mm (Kikusui-sha's RT-15 Model), whereby tablets (containing 20 mg/tablet of the

active ingredient) are obtained.

| Formulation: | |
|---|---|
| Compound 18 | 20 mg |
| Lactose | 143.4 mg |
| Potato starch | 30 mg |
| Hydroxypropyl cellulose | 6 mg |
| Magnesium stearate | 0.6 mg |
| | 200 mg |

Preparation Example 2: Injection preparation (Compound 16)
In a conventional method, an injection preparation having the following composition is prepared. Compound 16 (1 g) and D-mannitol (5 g) are added to distilled water for injection, and hydrochloric acid and aqueous sodium hydroxide solution are added thereto to adjust the pH to 6. Then distilled water for injection is added thereto to be 1000 mL in total. In a germ-free condition, the resulting mixture is filled into glass vials in an amount of 2 mL/vial, whereby an injection preparation (containing 2 mg/vial of the active ingredient) is obtained.
Formulation:

| | |
|---|---|
| Compound 16 | 2 mg |
| D-mannitol | 10 mg |
| Hydrochloric acid | ad lib. |
| Aqueous sodium hydroxide solution | ad lib. |
| Distilled water for injection | ad lib. |
| | 2.00 mL |

Industrial Applicability

According to the present invention, for example, thiazole derivatives or pharmaceutically acceptable salts thereof which have an adenosine $A_{2A}$ receptor antagonism and are useful for treating and/or preventing various diseases associated with adenosine $A_{2A}$ receptor (e.g., Parkinson's disease, Alzheimer's disease, depression, an ischemic disease such as cerebral infarction or myocardial infarction, etc.) and the like are provided.

## Claims

1. An adenosine $A_{2A}$ receptor antagonist comprising, as an active ingredient, a thiazole derivative represented by the general formula (I):

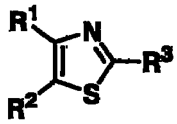

(I)

{wherein;
$R^1$ represents a substituted or unsubstituted 5-membered aromatic heterocyclic group containing at least one oxygen atom;
$R^2$ represents halogen,
substituted or unsubstituted lower alkyl,
substituted or unsubstituted lower alkenyl,
substituted or unsubstituted lower alkynyl,
substituted or unsubstituted cycloalkyl,
substituted or unsubstituted aryl,
substituted or unsubstituted aralkyl,

a substituted or unsubstituted alicyclic heterocyclic group,
a substituted or unsubstituted aromatic heterocyclic group,
substituted or unsubstituted alicyclic heterocyclic-alkyl,
substituted or unsubstituted aromatic heterocyclic-alkyl,
$-NR^4R^5$ (wherein
$R^4$ and $R^5$ may be the same or different, and each represents
a hydrogen atom,
substituted or unsubstituted lower alkyl,
substituted or unsubstituted lower alkenyl,
substituted or unsubstituted lower alkynyl,
substituted or unsubstituted lower alkanoyl,
substituted or unsubstituted cycloalkyl,
substituted or unsubstituted aryl,
substituted or unsubstituted aralkyl,
a substituted or unsubstituted alicyclic heterocyclic group,
a substituted or unsubstituted aromatic heterocyclic group,
substituted or unsubstituted alicyclic heterocyclic-alkyl, or
substituted or unsubstituted aromatic heterocyclic-alkyl), or
$-COR^6$ (wherein
$R^6$ represents substituted or unsubstituted lower alkyl,
substituted or unsubstituted lower alkenyl,
substituted or unsubstituted lower alkynyl,
substituted or unsubstituted cycloalkyl,
substituted or unsubstituted aryl,
substituted or unsubstituted aralkyl,
a substituted or unsubstituted alicyclic heterocyclic group,
a substituted or unsubstituted aromatic heterocyclic group,
substituted or unsubstituted alicyclic heterocyclic-alkyl, or
substituted or unsubstituted aromatic heterocyclicalkyl); and
$R^3$ represents $-NHCOR^7$ [wherein
$R^7$ represents $-NR^8R^9$ (wherein
$R^8$ and $R^9$ may be the same or different, and each represents
a hydrogen atom,
substituted or unsubstituted lower alkyl,
substituted or unsubstituted lower alkenyl,
substituted or unsubstituted lower alkynyl,
substituted or unsubstituted lower alkanoyl,
substituted or unsubstituted lower alkoxy,
substituted or unsubstituted cycloalkyl,
substituted or unsubstituted aryl,
substituted or unsubstituted aralkyl,
substituted or unsubstituted aroyl,
a substituted or unsubstituted alicyclic heterocyclic group,
a substituted or unsubstituted aromatic heterocyclic group,
substituted of unsubstituted alicyclic heterocyclicalkyl, or
substituted or unsubstituted aromatic heterocyclic-alkyl, or
$R^8$ and $R^9$ are combined together with the adjacent nitrogen atom thereto to form a substituted or unsubstituted alicyclic heterocyclic group) or
$-OR^{10}$ (wherein
$R^{10}$ represents substituted or unsubstituted lower alkyl,
substituted or unsubstituted lower alkenyl,
substituted or unsubstituted lower alkynyl,
substituted or unsubstituted cycloalkyl,
substituted or unsubstituted aryl,
substituted or unsubstituted aralkyl,
a substituted or unsubstituted alicyclic heterocyclic group,
a substituted or unsubstituted aromatic heterocyclic group,
substituted or unsubstituted alicyclic heterocyclic-alkyl, or

substituted or unsubstituted aromatic heterocyclic-alkyl)],
-CONHR$^{11}$ (wherein
R$^{11}$ represents a hydrogen atom,
substituted or unsubstituted lower alkyl,
substituted or unsubstituted lower alkenyl,
substituted or unsubstituted lower alkynyl,
substituted or unsubstituted lower alkanoyl,
substituted or unsubstituted cycloalkyl,
substituted or unsubstituted aryl,
substituted or unsubstituted aralkyl,
substituted or unsubstituted aroyl,
a substituted or unsubstituted alicyclic heterocyclic group,
a substituted or unsubstituted aromatic heterocyclic group,
substituted or unsubstituted alicyclic heterocyclic-alkyl, or
substituted or unsubstituted aromatic heterocyclic-alkyl), or
the general formula (II):

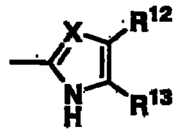

(II)

(wherein
=X- represents =CH- or =N-,
R$^{12}$ and R$^{13}$ may be the same or different, and each represents
a hydrogen atom,
halogen,
hydroxy,
nitro,
azido,
amino,
cyano,
carboxy,
formyl,
substituted or unsubstituted lower alkyl,
substituted or unsubstituted lower alkenyl,
substituted or unsubstituted lower alkynyl,
substituted or unsubstituted lower alkanoyl,
substituted or unsubstituted lower alkoxy,
substituted or unsubstituted cycloalkyl,
substituted or unsubstituted lower alkylamino,
substituted or unsubstituted di-lower alkylamino,
substituted or unsubstituted aryl,
substituted or unsubstituted aralkyl,
substituted or unsubstituted aroyl,
a substituted or unsubstituted alicyclic heterocyclic group,
a substituted or unsubstituted aromatic heterocyclic group,
substituted or unsubstituted alicyclic heterocyclic-alkyl, or
substituted or unsubstituted aromatic heterocyclic-alkyl, or
R$^{12}$ and R$^{13}$ are combined with the two carbon atoms adjacent thereto, respectively, to form
a substituted or unsubstituted carbon ring, or
a substituted or unsubstituted heterocyclic ring))}
or a pharmaceutically acceptable salt thereof.

**2.** The adenosine $A_{2A}$ receptor antagonist according to claim 1, wherein R$^1$ is substituted or unsubstituted furyl.

**3.** An agent for preventing and/or treating a disease associated with adenosine $A_{2A}$ receptor comprising, as an active ingredient, the thiazole derivative or the pharmaceutically acceptable salt thereof described in claim 1 or 2.

**4.** A thiazole derivative represented by the general formula (IA):

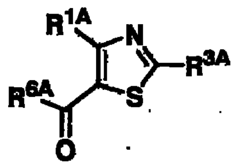

(IA)

(wherein $R^{1A}$, $R^{3A}$ and $R^{6A}$ have the same meanings as $R^1$, $R^3$ and $R^6$ defined above, respectively) or a pharmaceutically acceptable salt thereof.

**5.** The thiazole derivative or the pharmaceutically acceptable salt thereof according to claim 4, wherein $R^{1A}$ is substituted or unsubstituted furyl.

**6.** The thiazole derivative or the pharmaceutically acceptable salt thereof according to claim 4 or 5, wherein $R^{6A}$ is a substituted or unsubstituted alicyclic heterocyclic group, or a substituted or unsubstituted aromatic heterocyclic group, and -CO- in $-COR^{6A}$ binds to a carbon atom of $R^{6A}$.

**7.** The thiazole derivative or the pharmaceutically acceptable salt thereof according to any one of claims 4 to 6, wherein $R^{6A}$ is a substituted or unsubstituted alicyclic heterocyclic group.

**8.** The thiazole derivative or the pharmaceutically acceptable salt thereof according to any one of claims 4 to 6, wherein $R^{6A}$ is substituted or unsubstituted tetrahydropyranyl, substituted or unsubstituted pyridyl or substituted or unsubstituted dioxepanyl.

**9.** The thiazole derivative or the pharmaceutically acceptable salt thereof according to anyone of claims 4 to 8, wherein $R^{3A}$ is $-CONHR^{11}$ (wherein $R^{11}$ has the same meaning as defined above). ,

**10.** The thiazole derivative or the pharmaceutically acceptable salt thereof according to any one of claims 4 to 8, wherein $R^{3A}$ is the formula (II):

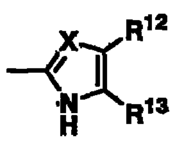

(II)

(wherein X, $R^{12}$ and $R^{13}$ have the same meanings as defined above, respectively).

**11.** The thiazole derivative or the pharmaceutically acceptable salt thereof according to claim 10, wherein $R^{12}$ and $R^{13}$ are combined with the two carbon atoms adjacent thereto, respectively, to form a substituted or unsubstituted carbon ring, or a substituted or unsubstituted heterocyclic ring.

**12.** The thiazole derivative or the pharmaceutically acceptable salt thereof according to any one of claims 4 to 8, wherein $R^{3A}$ is $-NHCOR^7$ (wherein $R^7$ has the same meaning as defined above).

**13.** A pharmaceutical composition comprising, as an active ingredient, the thiazole derivative or the pharmaceutically acceptable salt thereof described in any one of claims 4 to 12.

**14.** An adenosine $A_{2A}$ receptor antagonist comprising, as an active ingredient, the thiazole derivative or the pharmaceutically acceptable salt thereof described in any one of claims 4 to 12.

**15.** An agent for preventing and/or treating a disease associated with adenosine $A_{2A}$ receptor comprising, as an active Ingredient, the thiazole derivative or the pharmaceutically acceptable salt thereof described in any one of claims 4 to 12.

**16.** A method for antagonizing adenosine $A_{2A}$ receptor comprising the step of administering an effective amount of the thiazole derivative or the pharmaceutically acceptable salt thereof described in claim 1 or 2.

**17.** A method for preventing and/or treating a disease associated with adenosine $A_{2A}$ receptor comprising the step of administering an effective amount of the thiazole derivative or the pharmaceutically acceptable salt thereof described in claim 1 or 2.

**18.** A method for antagonizing adenosine $A_{2A}$ receptor comprising the step of administering an effective amount of the thiazole derivative or the pharmaceutically acceptable salt thereof described in any one of claims 4 to 12.

**19.** A method for preventing and/or treating a disease associated with adenosine $A_{2A}$ receptor comprising the step of administering an effective amount of the thiazole derivative or the pharmaceutically acceptable salt thereof described in any one of claims 4 to 12.

**20.** Use of the thiazole derivative or the pharmaceutically acceptable salt thereof described in claim 1 or 2 for the manufacture of an adenosine $A_{2A}$ receptor antagonist.

**21.** Use of the thiazole derivative or the pharmaceutically acceptable salt thereof described in claim 1. or 2 for the manufacture of an agent for preventing and/or treating adiseass associated with adenosine $A_{2A}$ receptor.

**22.** Use of the thiazole derivative or the pharmaceutically acceptable salt thereof described in any one of claims 4 to 12 for the manufacture of an adenosine An receptor antagonist.

**23.** Use of the thiazole derivative or the pharmaceutically acceptable salt thereof described in any one of claims 4. to 12 for the manufacture of an agent for preventing and/or treating adisease associated with adenosine $A_{2A}$ receptor.

**INTERNATIONAL SEARCH REPORT**

International application No.
PCT/JP2006/312619

A. CLASSIFICATION OF SUBJECT MATTER
*C07D417/14*(2006.01)i, *A61K31/427*(2006.01)i, *A61K31/437*(2006.01)i, *A61K31/438*(2006.01)i, *A61K31/4439*(2006.01)i, *A61K31/444*(2006.01)i, *A61K31/5377*(2006.01)i, *A61K31/55*(2006.01)i, *A61P3/10*(2006.01)i,

According to International Patent Classification (IPC) or to both national classification and IPC

B. FIELDS SEARCHED

Minimum documentation searched (classification system followed by classification symbols)
C07D417/14, A61K31/427, A61K31/437, A61K31/438, A61K31/4439, A61K31/444, A61K31/5377, A61K31/55, A61P3/10, A61P9/08, A61P9/10, A61P21/00, A61P21/04, A61P25/00, A61P25/02, A61P25/06, A61P25/08, A61P25/14, A61P25/16, A61P25/18,

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| P,X | WO 2005/63743 A1 (Kyowa Hakko Kogyo Co., Ltd.), 14 July, 2005 (14.07.05), Claims 1 to 50; table 5 (Family: none) | 1-8,12-15, 20-23 |
| A | JP 2002-302488 A (Takeda Chemical Industries, Ltd.), 18 October, 2002 (18.10.02), Full text & WO 01/74811 A2 & EP 1268474 A2 | 1-15,20-23 |
| A | WO 02/79204 A1 (Kyowa Hakko Kogyo Co., Ltd.), 10 October, 2002 (10.10.02), Full text (Family: none) | 1-15,20-23 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 August, 2006 (10.08.06) | 22 August, 2006 (22.08.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2006/312619

C (Continuation). DOCUMENTS CONSIDERED TO BE RELEVANT

| Category* | Citation of document, with indication, where appropriate, of the relevant passages | Relevant to claim No. |
|---|---|---|
| A | JP 2005-510508 A (Fujisawa Pharmaceutical Co., Ltd.),<br>21 April, 2005 (21.04.05),<br>Full text<br>& WO 03/39451 A2 & US 2005/4134 A1 | 1-15,20-23 |

Form PCT/ISA/210 (continuation of second sheet) (April 2005)

**INTERNATIONAL SEARCH REPORT**

International application No.
PCT/JP2006/312619

Continuation of A. CLASSIFICATION OF SUBJECT MATTER
(International Patent Classification (IPC))

*A61P9/08*(2006.01)i, *A61P9/10*(2006.01)i, *A61P21/00*(2006.01)i, *A61P21/04*(2006.01)i, *A61P25/00*(2006.01)i, *A61P25/02*(2006.01)i, *A61P25/06*(2006.01)i, *A61P25/08*(2006.01)i, *A61P25/14*(2006.01)i, *A61P25/16*(2006.01)i, *A61P25/18*(2006.01)i, *A61P25/20*(2006.01)i, *A61P25/22*(2006.01)i, *A61P25/24*(2006.01)i, *A61P25/28*(2006.01)i, *A61P25/30*(2006.01)i, *A61P31/18*(2006.01)i, *A61P43/00*(2006.01)i, *C07D471/04*(2006.01)i, *C07D491/08*(2006.01)i, *C07D491/107*(2006.01)i, *C07D493/08*(2006.01)i

(According to International Patent Classification (IPC) or to both national classification and IPC)

Continuation of B. FIELDS SEARCHED
Minimum documentation searched (International Patent Classification (IPC))

A61P25/20, A61P25/22, A61P25/24, A61P25/28, A61P25/30, A61P31/18, A61P43/00, C07D471/04, C07D491/08, C07D491/107, C07D493/08

Minimum documentation searched (classification system followed by classification symbols)

Form PCT/ISA/210 (extra sheet) (April 2005)

**INTERNATIONAL SEARCH REPORT**

International application No.
PCT/JP2006/312619

**Box No. II Observations where certain claims were found unsearchable (Continuation of item 2 of first sheet)**

This international search report has not been established in respect of certain claims under Article 17(2)(a) for the following reasons:

1. [x] Claims Nos.: 16-19
because they relate to subject matter not required to be searched by this Authority, namely:
Claims 16 to 19 are relevant to methods for treatment of the human body by surgery or therapy and diagnostic methods.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
[ ] The additional search fees were accompanied by the applicant's protest and, where applicable, payment of a protest fee..
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5314889 A
- US 5189049 A
- JP 2003335680 A
- JP 2002053566 A
- JP 11209284 A
- JP 10087490 A
- WO 9321168 A
- WO 9616650 A
- WO 9703058 A
- WO 0152847 A
- WO 0153267 A
- WO 0174811 A
- WO 02053156 A
- WO 02053161 A
- WO 02094798 A
- WO 03000257 A
- WO 03062215 A
- WO 03062233 A
- WO 03072554 A
- WO 03075923 A
- WO 2004002481 A
- WO 2004014884 A
- WO 2004041813 A
- WO 9921555 A
- JP 2001114779 A
- WO 9964418 A
- US 20040053982 A
- WO 03039451 A
- WO 2005039572 A
- US 6489476 B
- WO 0203978 A
- WO 0147935 A
- WO 0038666 A
- WO 0014095 A
- JP 11079993 A
- WO 9529904 A
- WO 9857937 A
- WO 0057877 A
- WO 02083111 A
- WO 03040147 A
- WO 2004016622 A
- WO 2004094395 A
- JP 63280255 A
- WO 9712615 A
- JP 5302680 A
- WO 0110865 A
- WO 0114372 A
- WO 02051442 A
- WO 02100433 A
- JP 5345772 A
- WO 02059099 A
- WO 0335639 A
- JP 11193281 A
- WO 03053925 A


### Non-patent literature cited in the description


- *European Journal of Pharmacology,* 1989, vol. 168, 285
- *Chemistry of Heterocyclic Compounds,* 2002, vol. 38, 873
- *Khimiko-Farmatsevticheskii Zhurnal,* 1974, vol. 8, 25
- *Journal of Medicinal Chemistry,* 1970, vol. 13, 638
- *Khimiya Geterotsiklicheskikh Soedinenii,* 1969, vol. 3, 498
- *Journal of Organic Chemistry,* 1962, vol. 27, 1351
- **T.W. GREENE.** Protective Groups in Organic Synthesis. John Wiley & Sons Inc, 1999
- **T. W. GREENE.** Protective Groups in Organic Synthesis. John Wiley & Sons Inc, 1981
- *Journal of the Chemical Society,* 1947, 114
- *Journal of the American Chemical Society,* 1953, vol. 72, 3722
- Lecture of Experimental Chemistry. Maruzen, 2003, vol. 15, 27
- Lecture of Experimental Chemistry. Maruzen, 1992, vol. 20, 473-483
- *Journal of the American Chemical Society,* 1954, vol. 76, 4458
- *Journal of Medicinal and Pharmaceutical Chemistry,* 1959, vol. 2, 588
- Lecture of Experimental Chemistry. Maruzen, 2003, vol. 15, 15
- *Journal of Organic Chemistry,* 1948, vol. 13, 722
- *Journal of Organic Chemistry,* 2002, vol. 67, 7551
- *Synlett,* 1992, 805

- *Synthetic Communications,* 2001, vol. 31, 3747
- *Journal of the Chemical Society,* 1949, vol. 71, 2473
- *Synthetic Communications,* 2000, vol. 30, 2287
- *Tetrahedron,* 1995, vol. 51, 5147
- *Journal of Chemical Society,* 1957, 2197
- *Journal of Organic Chemistry,* 1960, vol. 25, 1752
- *Molecular Pharmacology,* 1986, vol. 29, 331
- *The Journal of Pharmacology and Experimental Therapeutics,* 1989, vol. 251, 888
- *Journal of Neuroscience,* 1996, vol. 16, 605